(19) Europäisches Patentamt
European Patent Office
Office européen des brevets

(11) EP 0 499 926 B1

(12) EUROPÄISCHE PATENTSCHRIFT

(45) Veröffentlichungstag und Bekanntmachung des Hinweises auf die Patenterteilung:
**11.09.1996 Patentblatt 1996/37**

(51) Int Cl.[6]: **C07D 215/14**, A61K 31/47

(21) Anmeldenummer: **92102156.4**

(22) Anmeldetag: **10.02.1992**

(54) **2-Substituierte Chinoline, Verfahren zu ihrer Herstellung sowie ihre Verwendung in Arzneimitteln**

2-Substituted quinolines, process for their preparation as well as their use in medicaments

Quinolines 2-substituées, procédé pour leur préparation ainsi que leur utilisation comme médicaments

(84) Benannte Vertragsstaaten:
**AT BE CH DE DK ES FR GB GR IT LI LU NL PT SE**

(30) Priorität: **22.02.1991 DE 4105551**

(43) Veröffentlichungstag der Anmeldung:
**26.08.1992 Patentblatt 1992/35**

(73) Patentinhaber: **BAYER AG**
**51368 Leverkusen (DE)**

(72) Erfinder:
- **Raddatz, Siegfried, Dr.**
  **W-5000 Köln 80 (DE)**
- **Mohrs, Klaus-Helmut, Dr.**
  **W-5600 Wuppertal 1 (DE)**
- **Matzke, Michael, Dr.**
  **W-5600 Wuppertal 1 (DE)**
- **Fruchtmann, Romanis**
  **W-5000 Köln 1 (DE)**
- **Hatzelmann, Armin, Dr.**
  **W-5093 Burscheid (DE)**
- **Kohlsdorfer, Christian, Dr.**
  **W-5042 Erftstadt (DE)**
- **Müller-Peddinghaus, Reiner, Prof. Dr.**
  **W-5060 Bergisch Gladbach 2 (DE)**
- **Theisen-Popp, Pia, Dr.**
  **W-5100 Aachen (DE)**

(56) Entgegenhaltungen:
**EP-A- 0 339 416**
**EP-A- 0 344 519**
**EP-A- 0 399 291**
**EP-A- 0 414 076**
**EP-A- 0 414 078**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft 2-substituierte Chinoline, Verfahren zu ihrer Herstellung sowie ihre Verwendung in Arzneimitteln.

Substituierte 4-(Chinolin-2-yl-methoxy)phenylessigsäurederivate und α-substituierte 4-(Chinolin-2-yl-methoxy) phenylessigsäurederivate sind aus EP 344 519 (US 4 970 215) und EP 339 416 bekannt.
Darüber hinaus sind aus dem Stand der Technik weitere lipoxygenase inhibitorische Chinolin-methoxyphenyl-derivate bekannt, die sich im wesentlichen durch den Substituenten im Phenyl voneinander unterscheiden. So handelt es sich bei EP-A-0 399 291 um Essigsäuresulfonamide und in EP-A-0 414 076 sowie EP-A-0 414 078 um Essigsäurederivate, die in α-Stellung spiroverknüpft oder zusätzlich durch eine Hydroxyfunktion substituiert sind. Keine der in diesem Stand der Technik beschriebenen Verbindungen enthält einen zusätzlichen Substituenten in der Phenyleinheit.

Die vorliegende Erfindung betrifft nun 2-substituierte Chinoline der allgemeinen Formel (I)

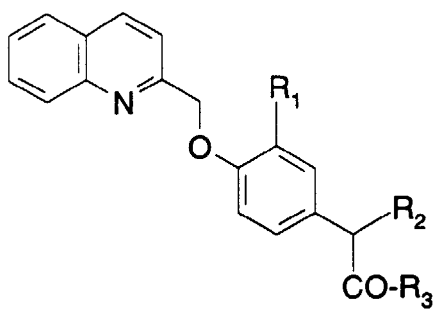

worin

$R^1$ für Fluor, Chlor, Brom, Iod, Cyano, Nitro, Azido, Trifluormethyl oder Trifluormethoxy steht, oder
für gradkettiges oder verzweigtes Alkoxy oder Acyl mit jeweils bis zu 6 Kohlenstoffatomen, oder
für geradkettiges oder verzweigtes Alkyl mit bis zu 6 Kohlenstoffatomen steht, das gegebenenfalls durch Hydroxy oder Alkyl mit bis zu 4 Kohlenstoffatomen substituiert ist, oder für geradkettiges oder verzweigtes Alkenyl mit bis zu 4 Kohlenstoffatomen steht, oder
für eine Gruppe der Formel $-NR^4R^5$ steht,
worin

$R^4$ und $R^5$ gleich oder verschieden sind und
Wasserstoff oder geradkettiges oder verzweigtes Alkyl mit bis zu 4 Kohlenstoffatomen bedeuten,

oder für Pyrryl, Pyridyl, Furyl oder Phenyl steht,

$R^2$ für Cycloalkyl mit 3 bis 12 Kohlenstoffatomen steht,

$R^3$ für einen Rest der Formel $-OR^6$ oder $NR^7-SO_2-R^8$ steht,
worin

$R^6$ Wasserstoff oder geradkettiges oder verzweigtes Alkyl mit bis zu 6 Kohlenstoffatomen bedeutet,

$R^7$ Wasserstoff oder geradkettiges oder verzweigtes Alkyl mit bis zu 4 Kohlenstoffatomen bedeutet,

$R^8$ Phenyl bedeutet, das gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann

und deren physiologisch unbedenklichen Salze.

Bevorzugt werden 5- und 6-gliedrige Heterocyclen mit bis zu 2 Stickstoffatomen wie beispielsweise Pyrryl, Pyrazolyl, Pyridyl, Pyrimidyl, Pyrazinyl oder Pyridazinyl, oder Furyl oder Thienyl genannt.

Im Rahmen der vorliegenden Erfindung werden physiologisch unbedenkliche Salze bevorzugt. Physiologisch unbedenkliche Salze der 2-substituierten Chinoline können Salze der erfindungsgemäßen Stoffe mit Mineralsäuren, Carbonsäuren oder Sulfonsäuren sein. Besonders bevorzugt sind z.B. Salze mit Chlorwasserstoffsäure,

Bromwasserstoffsäure, Schwefelsäure, Phosphorsäure, Methansulfonsäure, Ethan sulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I), in welcher

$R^1$ für Fluor, Chlor, Brom, Nitro, Azido oder Trifluormethoxy steht, oder
für geradkettiges oder verzweigtes Alkoxy oder Acyl mit jeweils bis zu 4 Kohlenstoffatomen steht, oder
für geradkettiges oder verzweigtes Alkyl mit bis zu 4 Kohlenstoffatomen steht, das gegebenenfalls durch Hydroxy oder Methoxy substituiert ist, oder für geradkettiges oder verzweigtes Alkenyl mit bis zu 4 Kohlenstoffatomen steht, oder
für eine Gruppe der Formel $-NR^4R^5$ steht,
worin

$R^4$ und $R^5$ gleich oder verschieden sind und
Wasserstoff oder geradkettiges oder verzweigtes Alkyl mit bis zu 3 Kohlenstoffatomen bedeuten,

oder für Pyrryl, Furyl oder Phenyl steht,

$R^2$ für Cyclobutyl, Cyclopentyl, Cyclohexyl, Cycloheptyl oder Cyclooctyl steht,

$R^3$ für einen Rest der Formel $-OR^6$ oder $-NR^7-SO_2-R^8$ steht,
worin

$R^6$ Wasserstoff oder geradkettiges oder verzweigtes Alkyl mit bis zu 4 Kohlenstoffatomen bedeutet,

$R^7$ Wasserstoff, Methyl oder Ethyl bedeutet,

$R^8$ Phenyl bedeutet, das gegebenenfalls durch Methyl, Fluor, Chlor, Brom, Jod, Methoxy oder Trifluormethyl substituiert ist, oder geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann

und deren physiologisch unbedenklichen Salze.

Außerdem wurden Verfahren zur Herstellung der erfindungsgemäßen Verbindungen der allgemeinen Formel (I) gefunden, dadurch gekennzeichnet, daß man

[A] im Fall, daß $R^3$ für die Gruppe $-OR^6$ steht
[$A_1$] entweder Verbindungen der allgemeinen Formel (IIa)

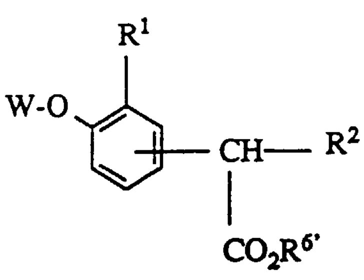

(IIa),

in welcher

$R^1$ und $R^2$ die oben angegebene Bedeutung haben,
W für eine Hydroxyschutzgruppe wie Benzyl oder tert.Butyl steht
und
$R^{6'}$ die oben angebene Bedeutung von $R^6$ aber nicht für Wasserstoff steht,
nach Abspaltung der Schutzgruppe mit 2-Halogenmethylchinolinen der allgemeinen Formel (III)

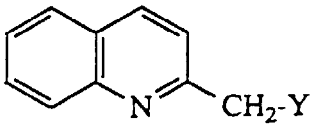

(III),

in welcher
Y für Halogen, insbesondere für Chlor oder Brom, steht,
in inerten Lösemitteln durch Veretherung in die Verbindungen der allgemeinen Formel (IVa)

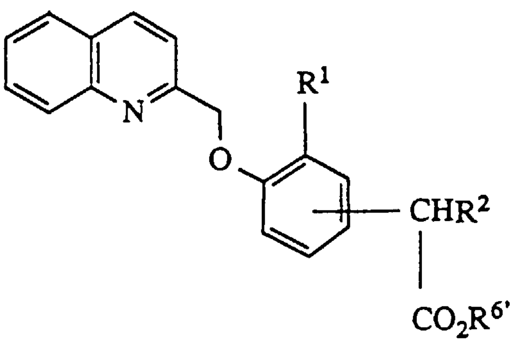

(IVa),

in welchen
$R^1$, $R^2$ und $R^{6'}$ die oben angegebene Bedeutung haben,
überführt und diese im Fall der Ester ($R^6 \neq H$) anschließend verseift,
oder
[$A_2$] Verbindungen der allgemeinen Formel (IIb)

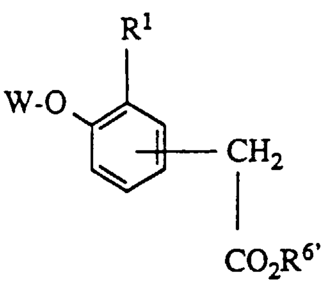

(IIb),

in welcher
$R^1$ und $R^{6'}$ die oben angegebene Bedeutung haben,
nach Abspaltung der Schutzgruppe zunächst mit 2-Halogenmethylchinolinen der allgemeinen Formel (III) in inerten Lösemitteln durch Veretherung in Verbindungen der allgemeinen Formel (IVb)

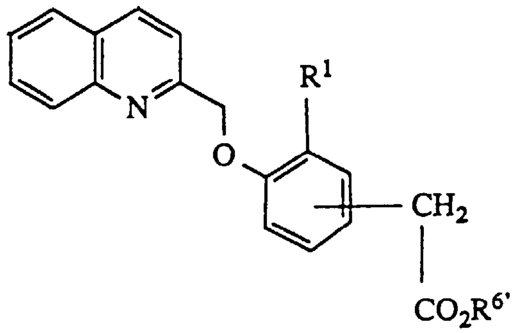

(IVb),

in welcher

$R^1$ und $R^{6'}$ die oben angegebene Bedeutung haben,

überführt,

und diese anschließend mit Verbindungen der allgemeinen Formel (V)

$$R^2\text{-}Z \qquad (V),$$

in welcher

$R^2$ die oben angegebene Bedeutung hat

und

Z für Chlor, Brom oder Iod steht,

in inerten Lösemitteln alkyliert und im Fall der Ester ($R^6 \neq H$) die Ester verseift,

oder

[B] im Fall, daß $R^3$ für die Gruppe $-NR^7-SO_2R^8$ steht,

Verbindungen der allgemeinen Formel (IVc)

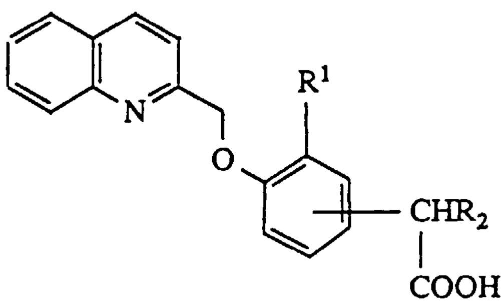

(IVc)

in welcher

$R^1$ und $R^2$ die oben angegebene Bedeutung haben,

in inerten Lösemitteln, gegebenenfalls in Anwesenheit einer Base mit Sulfonamiden der allgemeinen Formel (VI)

$$HNR^7\text{-}SO_2R^8 \qquad (VI),$$

in welcher

$R^7$ und $R^8$ die oben angegebene Bedeutung haben,

amidiert, und

[C] im Fall der Enantiomeren die entsprechenden enantiomerenreinen Säuren (IVc) nach üblicher Methode trennt und nach den oben aufgeführten Verfahren weiter umsetzt,

wobei der Substituent $R^1$ auf jeder der oben aufgeführten Stufen, gegebenenfalls nach üblichen chemischen Methoden, variiert werden kann.

Die erfindungsgemäßen Verfahren können durch folgendes Formelschema beispielhaft erläutert werden:

[A2]

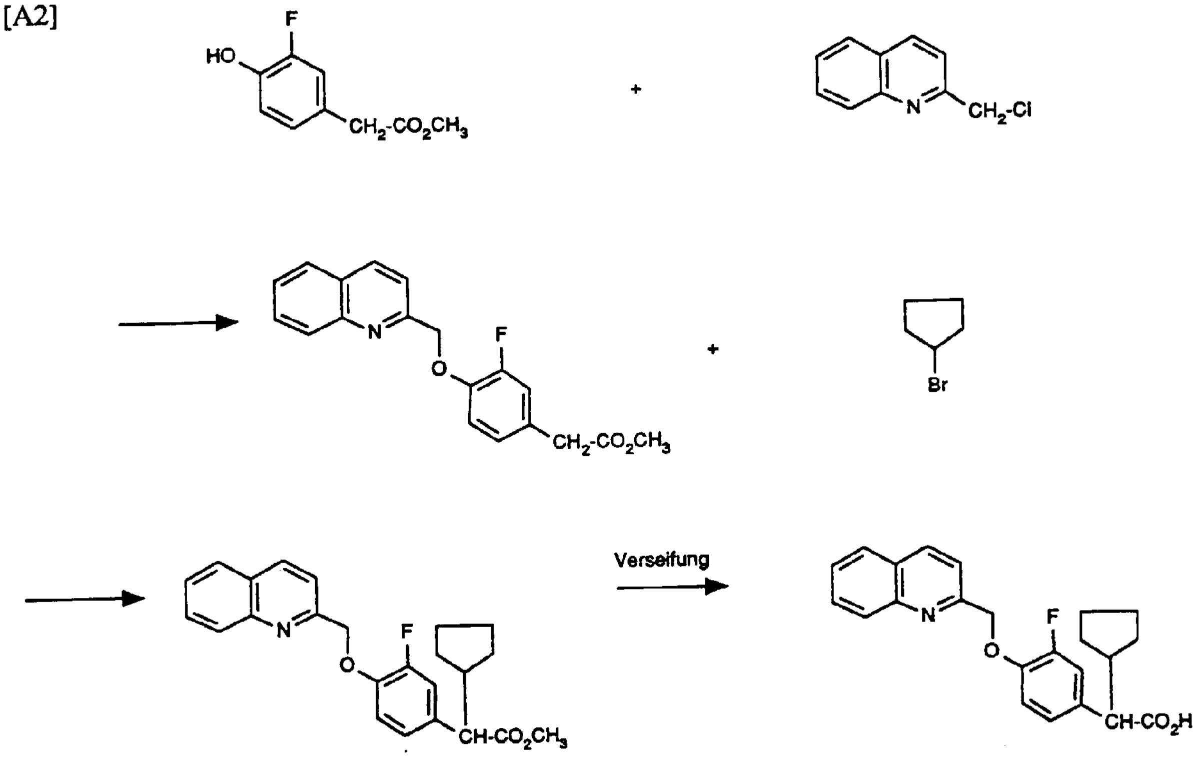


[A1]

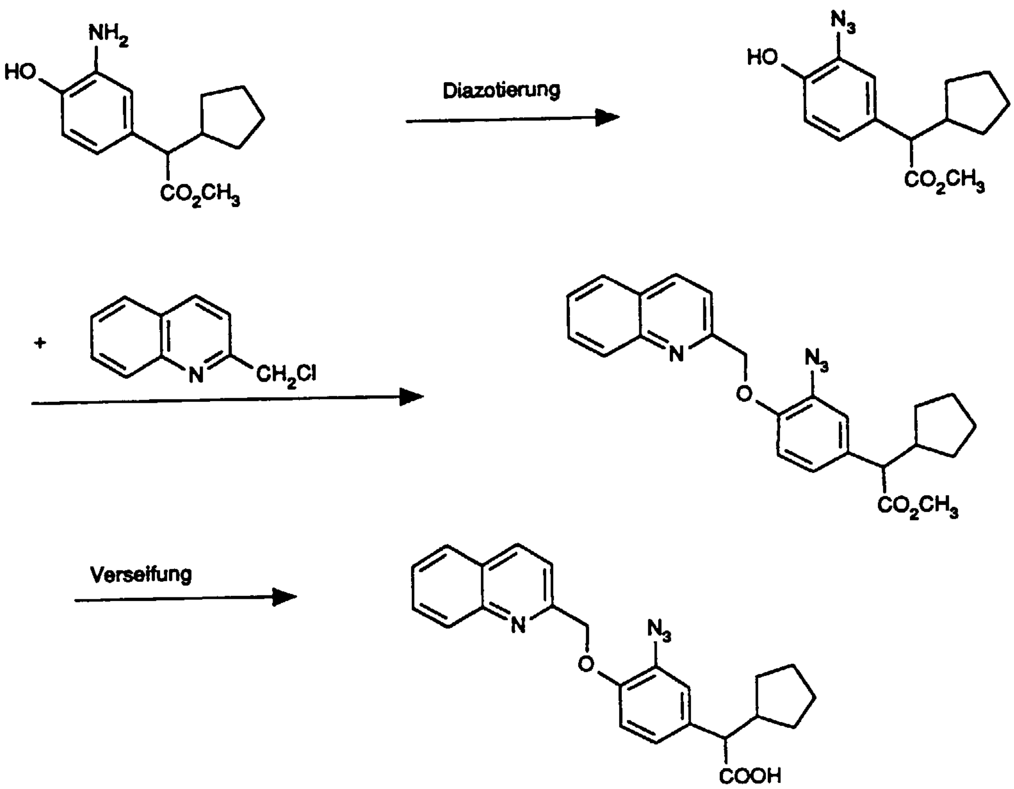

[B]

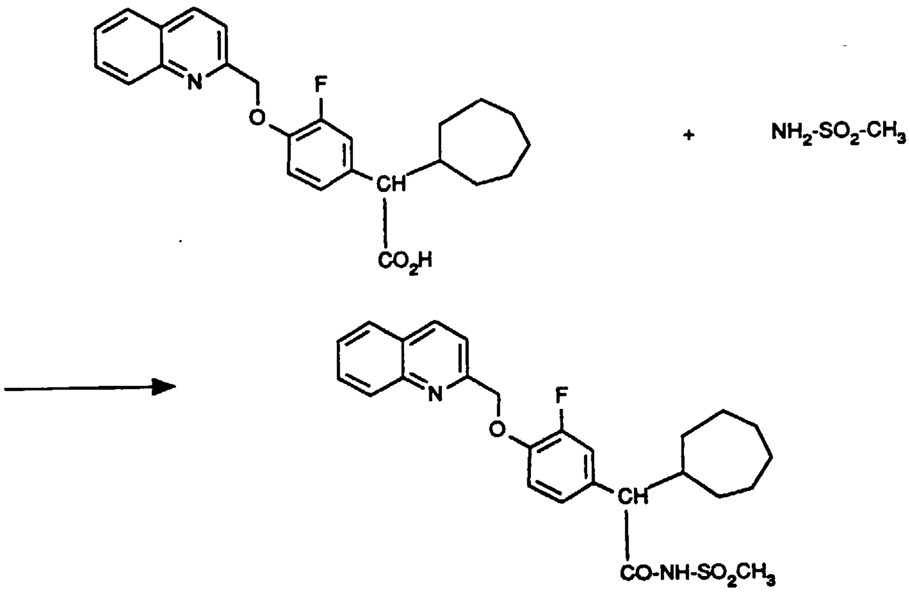


Die Abspaltung der Schutzgruppen aus den entsprechenden Ethern (IIa) und (IIb) erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Green: "Protective Groups in Organic Synthesis", J. Wiley & Sons, 1981, New York].

Die Veretherung kann in inerten organischen Lösemitteln gegebenenfalls in Anwesenheit einer Base durchgeführt werden. Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäurethamid. Ebenso ist es möglich, Gemische der Lösenmittel einzusetzen.

Als Basen für die Veretherung können anorganische oder organische Basen eingesetzt werden. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgefürht. Es ist aber auch möglich, das Verfahren bei Unterdruck oder Überdruck durchzuführen (z.B. in einem Bereich von 0,5 bis 5 bar).

Im allgemeinen setzt man 0,5 bis 5 Mol, bevorzugt 1 bis 2 Mol Halogenid (III), bezogen auf 1 Mol des Reaktionspartners, ein. Die Base wird im allgemeinen in einer Menge von 0,5 bis 5 Mol, bevorzugt von 1 bis 3 Mol, bezogen auf das Halogenid, eingesetzt.

Die Verbindungen der allgemeinen Formel (IIa) und (IIb) sind an sich bekannt oder können nach üblicher Methode hergestellt werden [vgl. J. Org. Chem. 31, 2658 (1966)].

Ebenfalls sind die Verbindungen der allgemeinen Formel (III) und ihre Darstellung bekannt [vgl. Chem. Ber. 120, 649 (1987)].

Als Lösemittel für die erfindungsgemäßen Verfahren und für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische

der genannten Lösemittel zu verwenden. Bevorzugt ist Dichlormethan.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgefürht. Es ist aber auch möglich, das Verfahren bei Unterdruck oder bei Überdruck durchzuführen (z.B. in einem Bereich von 0,5 bis 5 bar).

Bei der Durchführung der Amidierung wird die Base im allgemeinen in einer Menge von 1 bis 3 Mol, bevorzugt von 1 bis 1,5 Mol, bezogen auf 1 Mol der Carbonsäure (VIc), eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäurearhydrid oder Isobutylchloroformat oder Benzotriazolyloxy-tris-(dimethylamino)phosphonium-hexafluorophosphat oder Aminophosphonsäurediphenylester oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Frerman et al., J. Biol. Chem. 225, 507 (1982) und N.B. Beno iton, K. Kluroda, Int. Pept. Prot. Res. 13, 403 (1979), 17, 197 (1981)].

Die Verbindungen der allgemeinen Formeln (IVa), (IVb) und (IVc) sind neu und können nach der oben aufgeführten Methode hergestellt werden.

Die Verbindungen der allgemeinen Formel (V) sind bekannt [vgl. Beilstein 5,19/ 5,24/ 5,29] oder können nach üblichen Methoden aus den entsprechenden Alkoholen oder Cycloalkenen hergestellt werden.

Die Verbindungen der allgemeinen Formel (VI) sind bekannt [vgl. z.B. Beilstein 11/104].

Die erfindungsgemäßen phenylsubstituierten Chinoline können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysem, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Angina pectoris, Arteriosklerose, bei Gewebstransplatationen, Dermatosen wie Psoriasis, entzündliche Dermatosen und zur Cytoprotection im Gastrointestinaltrakt geeignet.

Die erfindungsgemäßen phenylsubstituierten Chinoline können sowohl in der Humanmedizin als auch in der Veterinärmedizin angewendet werden.

Die pharmakologischen Wirkungen der erfindungsgemäßen Substanzen werden durch folgende Methode bestimmt:

Als Maß für die Lipoxyenase-Hemmung wurde die Freisetzung von Leukotrien $B_4$ ($LTB_4$) an polymorphkernigen Humanleukozyten (PMN) nach Zugabe von Substanzen und Ca-Ionophor mittels reverse phase HPLC nach Borgeat, P. et al., Proc. Nat. Acad. Sci, 76, 2148 -2152 (1979), bestimmt.

In der Tabelle 1 sind beispielhaft die nach diesem Test erzielten Werte einiger erfindungsgemäßer Verbindungen aufgeführt:

Tabelle 1

| Beispiel-Nr. | 5-LO $IC_{50}$(µmol/l) |
|---|---|
| 1 | 2,50 |

Tabelle 1 (fortgesetzt)

| Beispiel-Nr. | 5-LO $IC_{50}$(μmol/l) |
|---|---|
| 27 | 0,69 |
| 40 | 0,79 |
| 41 | 0,56 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

3-Fluor-4-hydroxyphenylessigsäuremethylester

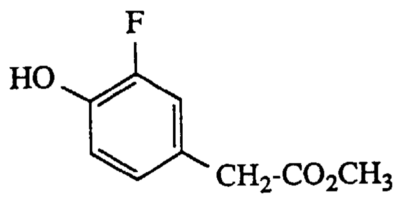

19,8 g (0,116 mol) 3-Fluor-4-hydroxyphenylessigsäure werden in 100 ml Methanol gelöst, mit 1 ml konz. Schwefelsäure versetzt und 2 h zum Sieden erhitzt. Nach dem Erkalten dampft man i.V. das Lösemittel ab, nimmt in 250 ml Dichlormethan auf und schüttelt 2 mal mit gesättigter $NaHCO_3$-Lösung aus. Nach Trocknen wird im Vakuum zur Trockene eingedampft, und man erhält ein zähes bernsteinfarbenes Öl.
Ausbeute: 18,4 g (85,8% der Theorie)

In Analogie zur Vorschrift des Beispiels I werden die in Tabelle I aufgeführten Beispiele hergestellt:

Tabelle I:

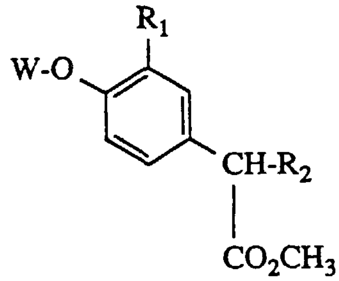

| Bsp.-Nr. | W | $R^1$ | $R^2$ | F°C | Ausbeute (%) |
|---|---|---|---|---|---|
| II | H | Br | H | Öl | 82,0 |
| III | H | $NO_2$ | H | 147 | quantitativ |

Beispiel IV

2-(4-Methallyloxyphenyl)-2-cyclopentyl-essigsäuremethylester

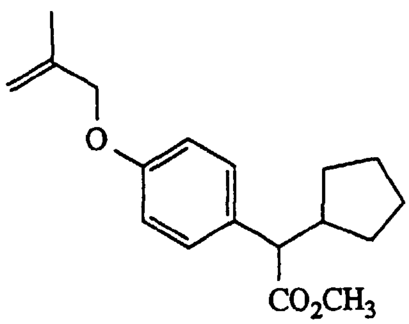

10 g (0,043 mol) 2-(4-Hydroxyphenyl)-2-cyclopentyl-essigsäuremethylester werden in 200 ml Dimethylformamid gelöst und unter Rühren mit 4,1 g (0,043 mol) Methallylchlorid und 5,9 g (0,043 mol) Kaliumcarbonat versetzt Man läßt über Nacht bei 100°C reagieren. Nach dem Erkalten dampft man das Lösemittel im Vakuum ab, nimmt den Rückstand in 200 ml Dichlormethan auf, wäscht zweimal mit 100 ml Wasser, trocknet die organische Phase mit Natriumsulfat und reinigt das im Vakuum eingedampfte Produkt säulenchromatographisch (Kieselgel 60, Laufmittel: Toluol/Essigsäureethylester = 100:5).
Ausbeute: 7,66 g (61,9% der Theorie) hellgelbes Öl

Beispiel V

2-(4-Hydroxy-3-methallylphenyl)-2-cyclopentyl-essigsäuremethylester

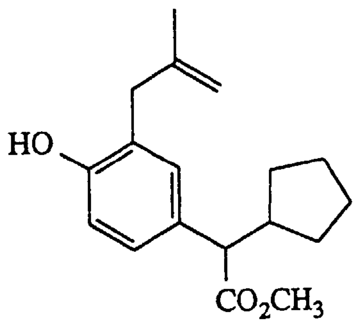

7,6 g (0,026 mol) der Verbindung aus Beispiel IV werden in 50 ml frisch destilliertem Diethylanilin gelöst und über Nacht auf 200°C erwärmt (Claisen-Umlagerung). Nach dem Erkalten destilliert man im Vakuum das Lösemittel ab, nimmt in 200 ml Dichlormethan auf und wäscht zweimal mit 40 ml 2 n Salzsäure, um Reste des Diethylanilins zu extrahieren. Anschließend wäscht man neutral, trocknet mit Natriumsulfat und konzentriert auf ein kleines Volumen. Die Reinigung erfolgt säulenchromatographisch (Kieselgel 60, Laufmittel: Toluol/Essigsäureethylester = 9:1).
Ausbeute: 4,3 g (57,4% der Theorie) hellgelbes Öl.

Beispiel VI

2-(4-Hydroxy-3-isobutylphenyl)-2-cyclopentyl-essigsäuremethylester

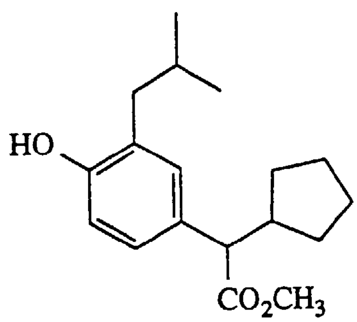

4 g (0,014 mol) der Verbindung aus Beispiel V werden in 30 ml Methanol und 10 ml Essigsäure gelöst und bei 5,3 bar mit Pd/C als Katalysator hydriert. Reaktionszeit: 2,5 h. Nach Abfiltrieren des Katalysators dampft man das Lösemittel im Vakuum ab und erhält ein leicht gelbliches Öl.
Ausbeute: 3,6 g (88,7% der Theorie)

Beispiel VII

4-Acetoxy-phenylessigsäuremethylester

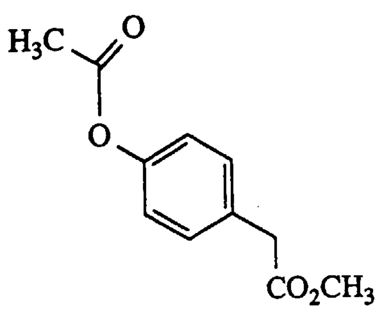

10 g (0,06 mol) 4-Hydroxyphenylessigsäuremethylester werden mit 18,36 g (0,18 mol) Acetanhydrid (17 ml) und 1 ml Pyridin versetzt und 2 Stunden zum Sieden erhitzt. Man dampft die Lösemittel im Vakuum weitgehend ab, nimmt in Wasser auf und extrahiert mit Essigsäureethylester. Nach Trocknen mit Natriumsulfat destilliert man das Lösemittel im Vakuum ab und erhält ein hellgelbes, dünnflüssiges Öl.
Ausbeute: 12,3 g (98,6% der Theorie)

Beispiel VIII

4-Hydroxy-3-acetyl-phenylessigsäuremethylester

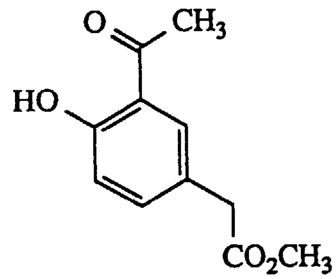

Unter Argon werden 5,3 g Aluminiumchlorid vorgelegt, mit 4 g (0,019 mol) der Verbindung aus Beispiel VII versetzt und 2 Stunden auf 150°C erwärmt (Friessche Verschiebung). Nach dem Erkalten versetzt man mit 50 ml Dichlortmethan, erwärmt kurz zum Sieden und filtriert. Die Reinigung erfolgt säulenchromatographisch (Kieselgel 60, Laufmittel: Toluol/Essigester = 8:2).
Ausbeute: 2,4 g (60.7% der Theorie) gelbes Öl.

In Analogie zur Vorschrift des Beispiels VII werden die in Tabelle II aufgeführten Beispiele hergestellt:

Tabelle II:

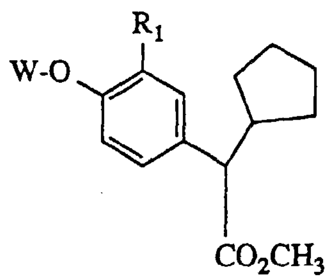

| Bsp.-Nr. | W | $R^1$ | F°C | Ausbeute (%) |
|---|---|---|---|---|
| IX | $H_3C$-CO- | H | Öl | 86,8 |
| X | H | $H_3C$-CO- | Öl | 96,0 |

Beispiel XI

2-(4-Hydroxy-3-nitrophenyl)-2-cyclopentyl-essigsäuremethylester

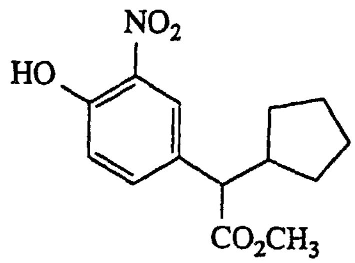

22,9 g (0,1 mol) 2-(4-Hydroxyphenyl)-2-cyclopentyl-essigsäuremethylester werden in 50 ml $CH_2Cl_2$ gelöst und bei 5°C zu einer Lösung von 50 ml konz. $HNO_3$/50 ml $H_2O$ zugetropft Es wird 15 min. gerührt, anschließend 100 ml $H_2O$ zugegeben und die organische Phase abgetrennt. Die wäßrige Phase wird dreimal mit 50 ml $CH_2Cl_2$ extrahiert, die organischen Phasen werden 5 mal mit Wasser gewaschen, getrocknet, auf ein kleines Volumen eingeengt und über Kieselgel filtriert. Nach Einengen erhält man das Produkt in 71%iger Ausbeute (20 g). Das Produkt wird roh weiterverarbeitet.

Beispiel XII

2-(3-Amino-4-hydroxy-phenyl)-2-cyclopentylessigsäuremethylester

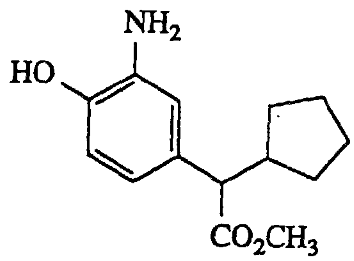

5,6 g (20 mmol) der Verbindung aus Beispiel XI werden in 100 ml Ethanol unter Zusatz von 0,5 g Palladium/Kohle (10%ig) bei 4 atm. hydriert. Der Katalysator wird abgesaugt, das Filtrat eingeengt und ohne weitere Reinigung weiter umsetzt (Ausbeute quantitativ).

Beispiel XIII

2-(3-Azido-4-hydroxy-phenyl)-2-cyclopentyl-essigsäuremethylester

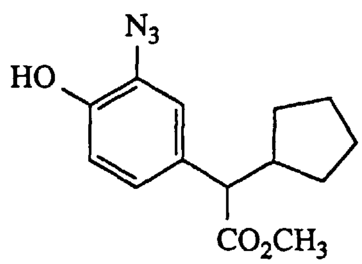

5,0 g (20 mmol) des Rohproduktes aus Beispiel XII werden in 20 ml $H_2O$, 10 ml Ethanol und 20 ml konz. HCl gelöst und bei 0°C mit 1,8 g (26 mmol) Natriumnitrit in 10 ml $H_2O$ diazotiert. Nach beendeter $N_2$-Entwicklung wird dreimal mit 100 ml $CH_2Cl_2$ extrahiert, die organischen Phasen werden eingeengt und über Kieselgel 60 ($CH_2Cl_2$/MeOH = 100: 2) chromatographiert.
Ausbeute: 4,5 g (82% der Theorie)
Fp.: 59-60°C

Beispiel XIV

2-[3-Fluor-4-(Chinolin-2-yl-methoxy)phenyl]essigsäuremethylester

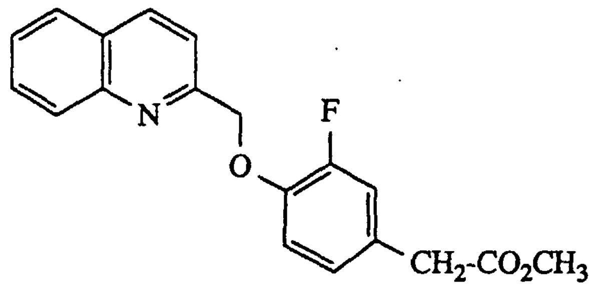

18,4 g (0,1 mol) der Verbindung aus Beispiel I werden in 50 ml DMF gelöst und mit 4 g (0,1 mol) NaOH in 40 ml Methanol versetzt Zu dieser Mischung läßt man unter Rühren 17,8 g (0,1 mol) 2-Chlormethylchinolin in 50 ml DMF tropfen und erwärmt anschließend 5 h auf 100°C. Nach dem Abkühlen dampft man im Vakuum das Lösemittel ab, nimmt in Dichlormethan auf, wäscht zweimal mit Wasser, trocknet und engt im Vakuum auf ein kleines Volumen ein. Es erfolgt eine säulenchromatographische Trennung (Kieselgel 60, Laufmittel: Toluo/Essigester = 9:1 bis 8:2).
Ausbeute: 28 g (86% der Theorie) gelbes Öl.

Beispiel XV

2-[3-Fluor-4-(Chinolin-2-yl-methoxy)phenyl]-essigsäure

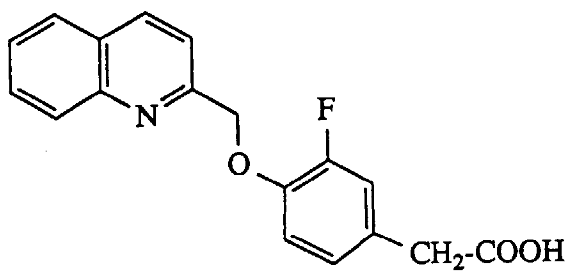

25 g (0,077 mol) der Verbindung aus Beispiel XIV werden in 300 ml Methanol gelöst und mit 125 ml 1 molarer Natronlauge versetzt Man rührt 3 h zum Sieden, läßt erkalten und neutralisiert mit 1 n Salzsäure. Man dampft alles im Vakuum zur Trockene ein, übergießt mit 50 ml Wasser und mit 150 ml Dichlormethan. Die Dichlormethanphase trocknet man und dampft im Vakuum das Lösemittel ab. Zurück bleiben farblose Kristalle.
Ausbeute: 19,5 g (81,5% der Theorie)
Fp.: 177-179°C

Beispiel XVI

2-[3-Fluor4-(chinolin-2-yl-methoxy)phenyl]-acetyl-methansulfonamid

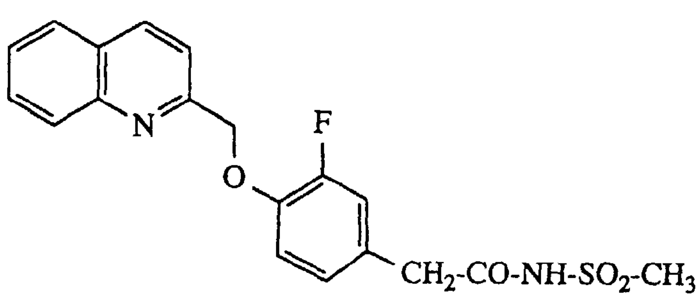

6 g (0,019 mol) der Verbindung aus Beispiel XV, 1,9 g (0,019 mol) getrocknetes Methansulfonamid, 3,8 g (0,019 mol) N-Ethyl-N'-dimethylaminopropylcarbodiimid-hydrochlorid und 2,4 g (0,019 mol) Dimethylaminopyridin werden in 40 ml Dichlormethan gelöst und bei Raumtemperatur 60 h gerührt. Anschließend dampft man im Vakuum zur Trockene ein, nimmt in 40 ml Dichlormethan auf und wäscht zweimal mit 20 ml Wasser. Nach Trocknen der organischen Phase mit $Na_2SO_4$ dampft man im Vakuum ein und trennt den Rest säulenchromatographisch (Kieselgel 60, Laufmittel Dichlormethan/Essigester/Eisessig = 10/1/1).

Ausbeute: 5,2 g (70,5% der Theorie) farbl. Kristalle
Fp.: 171°C

Beispiel XVII

2-[3-Fluor-4-(chinolin-2-yl-methoxy)phenyl]-acetyl-benzylsulfonamid

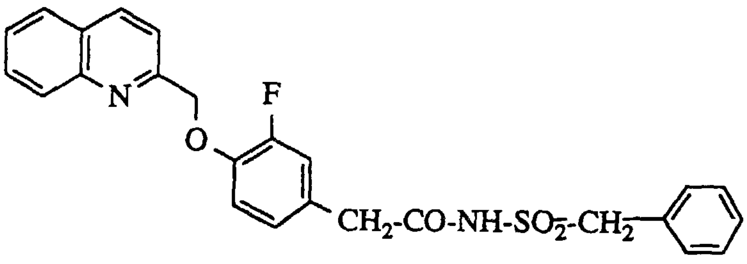

In Analogie zu Beispiel XVI wird die Titelverbindung aus 4 g (0,013 mol) der Verbindung aus Beispiel XV, 2,22 g (0,013 mol) getrocknetem Benzylsulfonamid, 2,49 g (0,013 mol) N-Ethyl-N'-dimethylaminopropyl-carbodiimid-hydrochlorid und 1,59 g (0,013 mol) Dimethylaminopyridin erhalten.
Ausbeute: 4,4 g (72,9% der Theorie) farbl. Kristalle
Fp.: 156°C

Beispiel XVIII

2-[3-Chlor-4-(chinolin-2-yl-methoxy)phenyl]-essigsäuremethylester

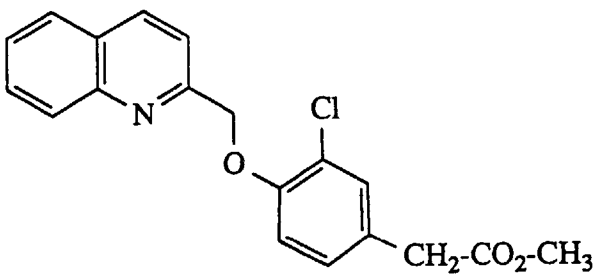

Die Titelverbindung wird in Analogie zur Vorschrift des Beispiels XIV aus 5,3 g (0,03 mol) 2-Chlormethylchinolin, 6 g (0,03 mol) 3-Chlor-4-hydroxyphenylessigsäuremethylester und 1,2 g (0,03 mol) Natriumhydroxid hergestellt.
Ausbeute: 8,7 g (84,9% der Theorie) farbl. Kristalle
Fp.: 79°C

Beispiel XIX

2-[3-Chlor-4-(Chinolin-2-yl-methoxy)phenyl]-essigsäure

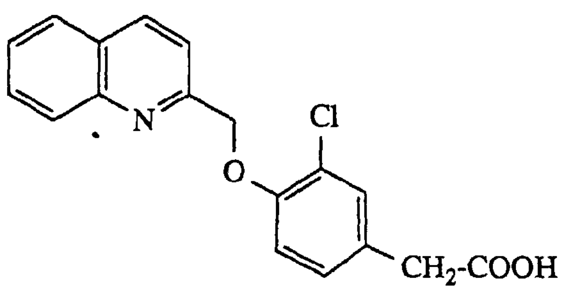

In Analogie zur Vorschrift des Beispiels XV wird die Titelverbindung aus 4 g (0,012 mol) der Verbindung aus Beispiel XVIII und 18 ml 1 n Natronlauge erhalten.

Ausbeute: 3,5 g (89,1% der Theorie) farbl. Kristalle
Fp.: 203-205°C

Beispiel XX

2-[3-Brom-4-(chinolin-2-yl-methoxy)phenyl]essigsäuremethylester

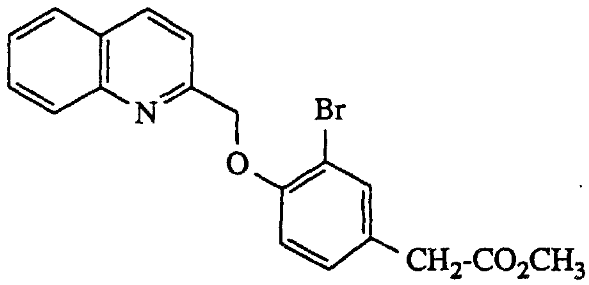

In Analogie zur Vorschrift des Beispiels XIV wird die Titelverbindung aus 17 g (0,07 mol) der Verbindung aus Beispiel II, 12,32 g (0,07 mol) 2-Chlormethylchinolin und 2,8 g (0,07 mol) Natriumhydroxid hergestellt.
Ausbeute: 23,2 g (85,8% der Theorie) leicht gelbl. Kristalle
Fp.: 90°C

Beispiel XXI

2-[3-Brom-4-(chinolin-2-yl-methoxy)phenyl]essigsäure

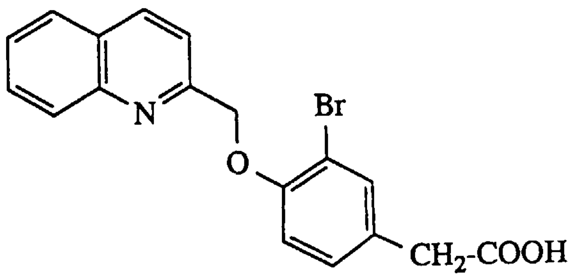

In Analogie zur Vorschrift des Beispiels XV wird die Titelverbindung aus 3 g (7,77 mmol) der Verbindung aus Beispiel XX und 12 ml 1 n Natronlauge (12 mmol) hergestellt.
Ausbeute: 2,5 g (86,5% der Theorie) farbl. Kristalle
Fp.: 206 - 208°C (Zers.)

Beispiel XXII

2-[3-Brom-4-(chinolin-2-yl-methoxy)phenyl]acetylmethansulfonamid

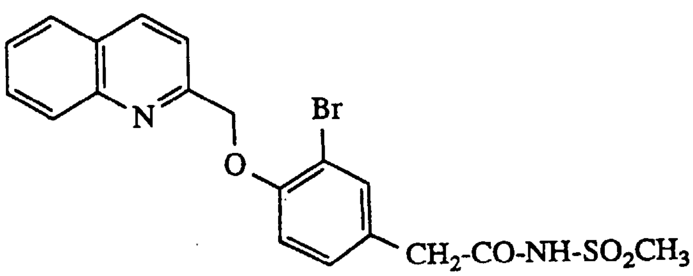

In Analogie zur Vorschrift des Beispiels XVI wird die Titelverbindung aus 2,8 g (7,5 mmol) der Verbindung aus Beispiel XXI, 0.71 g (7,5 mmol) getrocknetem Methansulfonamid, 1,44 g (7,5 mmol) N-Ethyl-N'-dimethylaminopropylcarbodiimid-hydrochlorid und 0,92 g (7,5 mmol) Dimethylaminopyridin hergestellt.

Ausbeute: 0,86 g (25,5% der Theorie) farbl. Kristalle
Fp.: 212°C (Zers.)

Beispiel XXIII

2-[3-Methoxy-4-(chinolin-2-yl-methoxy)phenyl]essigsäure-methylester

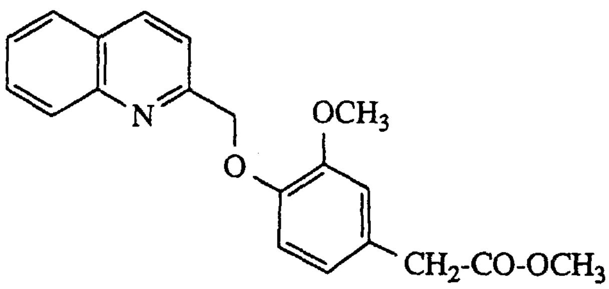

In Analogie zur Vorschrift des Beispiels XIV wird die Titelverbindung aus 16 g (0,082 mol) 3-Methoxy-4-hydroxyphenylessigsäuremethylester, 14,5 g (0,082 mol) 2-Chlormethylchinolin und 3,28 g (0,082 mol) Natriumhydroxid hergestellt.
Ausbeute: 20,5 g (74,1% der Theorie) farbl. Kristalle
Fp.: 69°C

Beispiel XXIV

2-[3-Methoxy-4-(chinolin-2-yl-methoxy)phenyl]-essigsäure

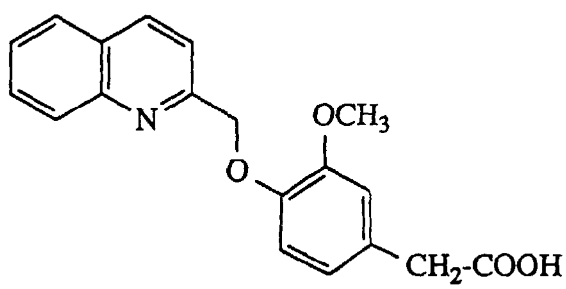

Analog zur Vorschrift des Beispiels XV wird die Titelverbindung aus 3 g (8,9 mmol) der Verbindung aus Beispiel XXIII und 12 ml 1 n Natronlauge hergestellt.
Ausbeute: 2,4 g (83,4% der Theorie) farbl. Kristalle
Fp.: 168-170°C (Zers.)

Beispiel XXV

2-[3-Trifluormethylthio-4-(chinolin-2-yl-methoxy)phenyl]essigsäure-ethylester

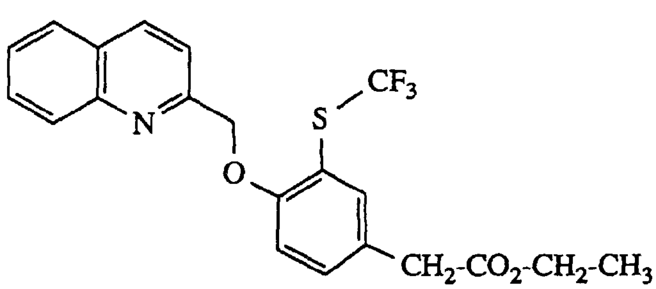

In Analogie zur Vorschrift des Beispiels XIV wird die Titelverbindung aus 10 g (0,036 mol) 4-Hydroxy-3-trifluormethylthiophenylessigsäureethylester, 7,7 g (0,036 mol) 2-Chlormethylchinolin und 2,88 g (0,072 mol) Natriumhydroxid hergestellt.
Ausbeute: 7,55 g (49,8% der Theorie) gelbes Öl.

Beispiel XXVI

2-[3-Trifluormethylthio-4-(chinolin-2-yl-methoxy)phenyl]essigsäure

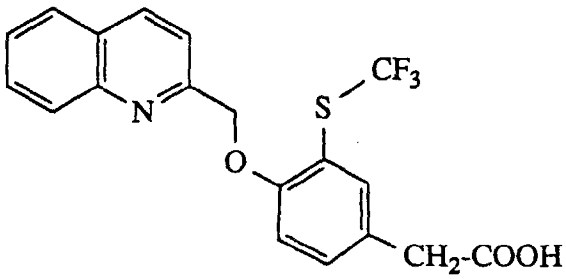

In Analogie zur Vorschrift des Beispiels XV wird die Titelverbindung aus 2,1 g (5 mmol) der Verbindung aus Beispiel XXV und 0,4 g (0,01 mol) Natriumhydroxid in Dioxan/Wasser hergestellt.
Ausbeute: 1,8 g (91,6% der Theorie) farbl. Kristalle
Fp.: 152°C

Beispiel XXVII

2-[3-Trifluormethylthio-4-(chinolin-2-yl-methoxy)phenyl]acetyl-methansulfonamid

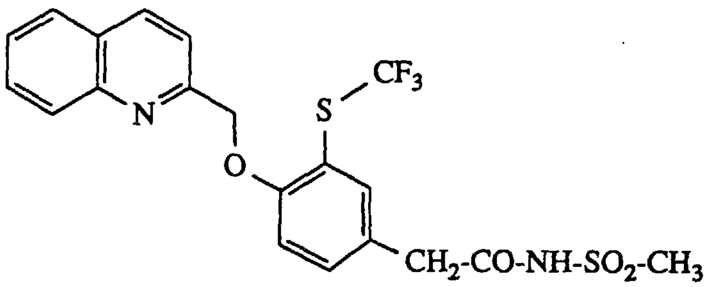

In Analogie zur Vorschrift des Beispiels XV wird die Titelverbindung aus 1,2 g (3,1 mmol) der Verbindung aus Beispiel XXVI, 0,38 g (4 mmol) Methansulfonamid, 0,77 g (4 mmol) N-Ethyl-N'-dimethylaminopropyl-carbodiimid-hydrochlorid und 0,49 g (4 mmol) Dimethylaminopyridin hergestellt.
Ausbeute: 1,2 g (82,4% der Theorie) farbl. Kristalle
Fp.: 183°C (Zers.)

Beispiel XXVIII

2-[3-Nitro-4-(chinolin-2-yl-methoxy)phenyl]essigsäuremethylester

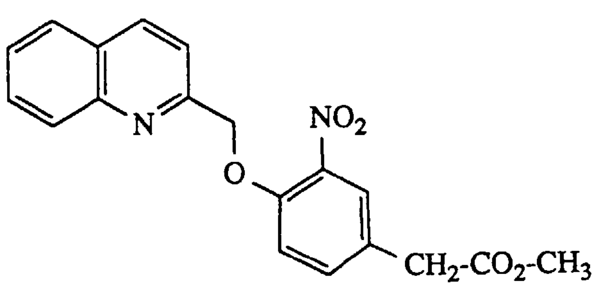

In Analogie zur Vorschrift des Beispiels XIV wird die Titelverbindung aus 10,75 g (0,0509 mol) der Verbindung aus Beispiel III, 10,9 g (0,051 mol) 2-Chlormethylchinolin und 4,32 g (0,11 mol) Natriumhydroxid hergestellt.
Ausbeute: 3,3 g (18,4% der Theorie) gelbe Kristalle

Fp.: 177°C

Beispiel XXIX

2-[3-Amin-4-(chinolin-2-yl-methoxy)phenyl]essigsäuremethylester

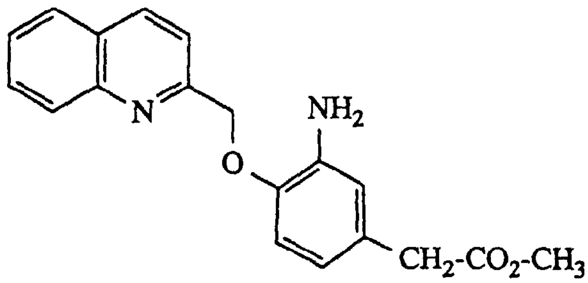

15 g (0,043 mol) der Verbindung aus Beispiel XXVIII werden in 100 ml Tetrahydrofuran und 100 ml Methanol gelöst und mit 4 g (0,08 mol) Hydrazinmonohydrat versetzt Unter Argon gibt man unter Rühren portionsweise Raney-Nickel hinzu, wobei die Temperatur auf 50°C ansteigt Nach Beendigung der Gasentwicklung erhitzt man noch eine Stunde zum Sieden und filtriert dann heiß. Das Filtrat wird im Vakuum eingeengt und das restliche Öl mit 250 ml Dichlormethan aufgenommen. Nach zweimaligem Waschen mit Wasser und Trocknen mit Natriumsulfat dampft man das Lösemittel im Vakuum ab und erhält ein farbloses Öl, das über Nacht kristallisiert.
Ausbeute: 12,5 g (90,3% der Theorie) farbl. Kristalle
Fp.: 75°C

Beispiel XXX

2-[3-(1-Pyrryl)4-(chinolin-2-yl-methoxy)phenyl]essigsäuremethylester

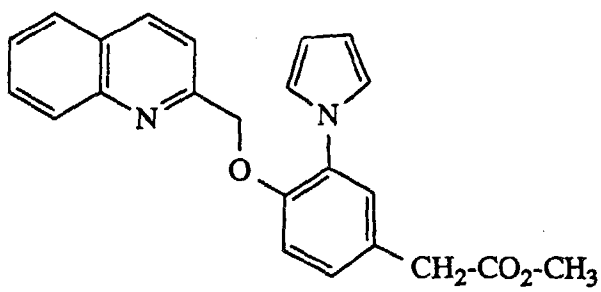

5 g (0,016 mmol) der Verbindung aus Beispiel XXIX werden in 70 ml Essigsäure gelöst, mit 2,78 g (0,02 mol) 2,5-Dimethoxytetrahydrofuran versetzt und 2 Stunden zum Sieden erhitzt. Nach Abdestillieren der Essigsäure im Vakuum, Aufnehmen in 200 ml Dichlormethan, Ausschütteln mit Wasser, Trocknen mit Natriumsulfat und Einengen im Vakuum auf ein kleines Volumen wird das verbleibende braune Öl (6 g) säulenchromatographisch getrennt (Kieselgel 60, Laufmittel: Toluol/Essigester = 4/1).
Ausbeute: 3,2 g (53,8% der Theorie) farbl. Kristalle
Fp.: 102°C

Beispiel XXXI

2-[3-(1-Pyrryl)-4-(chinolin-2-yl-methoxy)phenyl]essigsäure

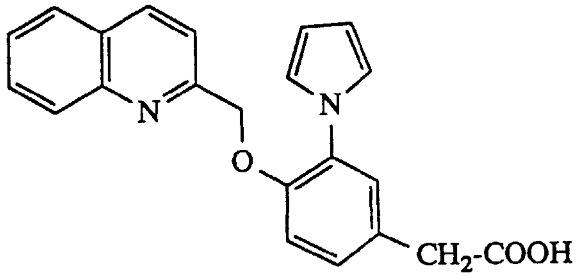

In Analogie zur Vorschrift des Beispiels XV wird die Titelverbindung aus 0,8 g (2 mmol) der Verbindung aus Beispiel XXX und 0,2 g (5 mmol) Natriumhydroxid in 50 ml Isopropanol hergestellt.
Ausbeute: 0,7 g (97,8% der Theorie) farbl. Kristalle
Fp.: 173°C

Beispiel XXXII

2-[3-Vinyl-4-(chinolin-2-yl-methoxy)phenyl]essigsäuremethylester

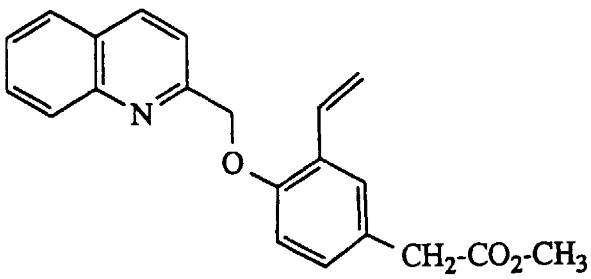

In einem 50 ml Braunglaskolben (mit Argon gespült) werden 200 mg (0,21 mmol) des Katalysators $[P(Phenyl)_3]_4Pd$ eingewogen und unter Argon mit 2 g (5,2 mmol) der Verbindung aus Beispiel XX und 1,4 ml (5,2 mmol) $Bu_3SnCH=CH_2$ (d = 1,086), beides in 10 ml Toluol gelöst, versetzt. In einer lichtgeschützten Apparatur erhitzt man das Gemisch unter Rühren 20 Stunden zum Sieden. Anschließend dampft man das Lösemittel im Vakuum ab und trennt den Rest säulenchromatographisch (Kieselgel 60, Laufmittel: Toluol/Essigsäureethylester = 4:1).
Ausbeute: 1,5 g (86,6% der Theorie) farbl. Kristalle
Fp.: 69°C

Beispiel XXXIII

2-[3-Vinyl-(chinolin-2-yl-methoxy)phenyl]essigsäure

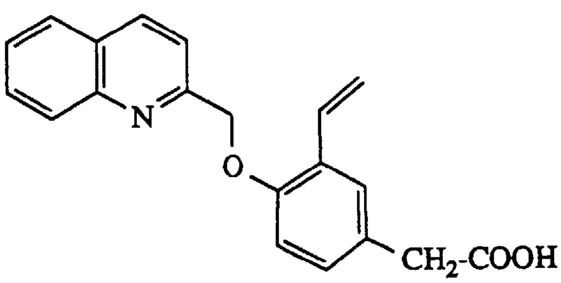

In Analogie zur Vorschrift des Beispiels XV wird die Titelverbindung aus 1,1 g (3,3 mmol) der Verbindung aus Beispiel XXXII und 5 ml (5 mmol) 1 n Natronlauge hergestellt.
Ausbeute: 1,0 g (95,0% der Theorie) farbl. Kristalle
Fp.: 173°C

Beispiel XXXIV

2-[3-Ethyl-4-(chinolin-2-yl-methoxy)phenyl]essigsäuremethylester

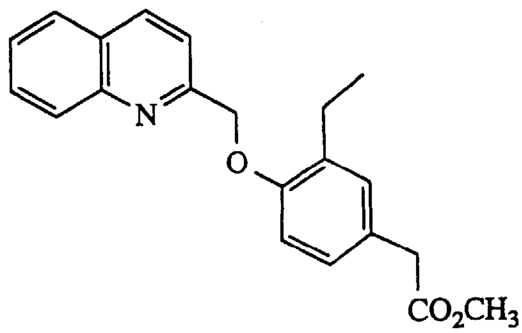

14,3 g (0,0429 mol) der Verbindung aus Beispiel XXXII werden in 150 ml Methanol und 15 ml Eisessig gelöst, mit 1,5 g Pd-C 5%ig versetzt, auf 30-35°C erwärmt und hydriert Der Reaktionsansatz wird über Kieselgel filtriert, das Filtrat im Vakuum eingeengt und der Rückstand aus Isopropanol umkristallisiert.
Ausbeute: 8,5 g (59,1% der Theorie) farbl. Kristalle
Fp.: 72°C

Beispiel XXXV

2-[3-Ethyl-4-(chinolin-2-yl-methoxy)phenyl]essigsäure

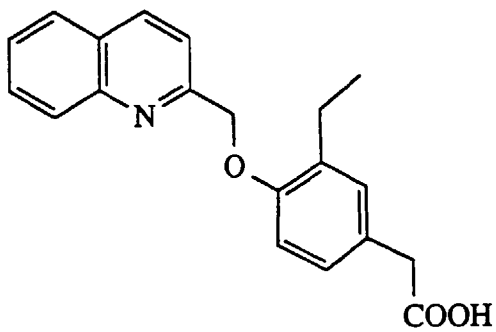

Analog zur Vorschrift des Beispiels V wird die Titelverbindung aus 1,5 g (4,48 mmol) der Verbindung aus Beispiel XXXIV und 10 ml (10 mmol) 1 n Natronlauge hergestellt.
Ausbeute: 1,4 g (97,4% der Theorie) farbl. Kristalle
Fp.: 144°C

Beispiel XXXVI

N-[3-Ethyl-4-(chinolin-2-yl-methoxy)phenyl]acetylmethansulfonamid

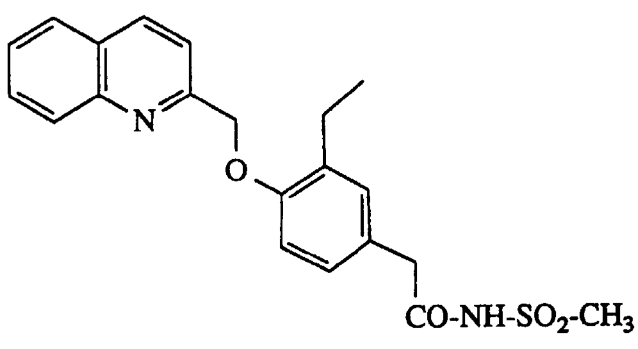

Analog zur Vorschrift des Beispiels XVI wird die Titelverbindung aus 1,7 g (5,3 mmol) der Verbindung aus Beispiel XXXV, 0,6 g (6 mmol) Methansulfonamid, 1,2 g (6 mmol) N-Ethyl-N'-dimethylaminopropylcarbodiimid-hydrochlorid und 0,8 g (6 mmol) Dimethylaminopyridin hergestellt.
Ausbeute: 1,4 g (66,3% der Theorie) farbl. Kristalle
Fp.: 170°C

Beispiel XXXVII

2-[3-Allyl-4-(chinolin-2-yl-methoxy)phenyl]essigsäuremethylester

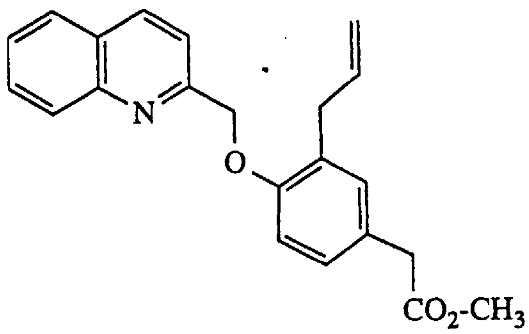

In Analogie zur Vorschrift des Beispiels XXXII wird die Titelverbindung aus 16,6 g (0,043 mol) der Verbindung aus Beispiel XX, 13,6 g (0,043 mol) $Bu_3$-Sn-$CH_2$-CH=$CH_2$ und 2,0 g (0,0017 mol) $[P(Phenyl)_3]_4Pd$ hergestellt.
Ausbeute: 7,6 g (50,9% der Theorie) farbl. Kristalle
Fp.: 71°C

Beispiel XXXVIII

2-[3-Allyl-4-(chülolin-2-yl-methoxy)phenyl]essigsäure

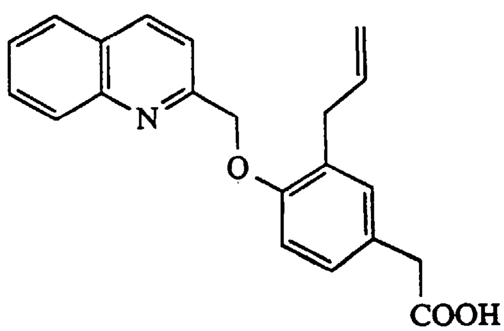

In Analogie zur Vorschrift des Beispiels XV wird die Titelverbindung aus 2,0 g (5,8 mmol) der Verbindung aus Beispiel XXXVII und 10 ml (10 mmol) 1 n Natronlauge hergestellt.
Ausbeute: 1,7 g (88,0% der Theorie) farbl. Kristalle
Fp.: 130°C

Beispiel XXXIX

2-[3-Propyl-4-(chinolin-2-yl-methoxy)phenyl]essigsäuremethylester

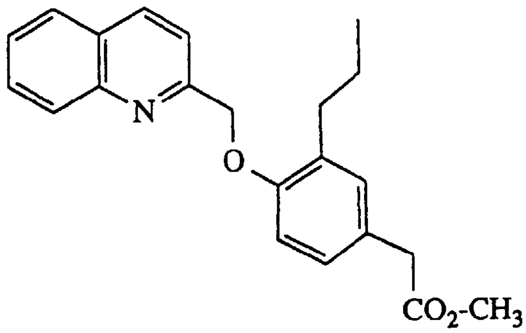

In Analogie zur Vorschrift des Beispiels XXXIV wird die Titelverbindung aus 7,5 g (0,0225 mol) der Verbindung aus Beispiel XXXVII und 0,8 g Pd/C (5%) mit Wasserstoff hergestellt.
Ausbeute: 6,5 g (82,8% der Theorie) gelbliches Öl

Beispiel XL

2-[3-Propyl-4-(chinolin-2-yl-methoxy)phenyl]essigsäure

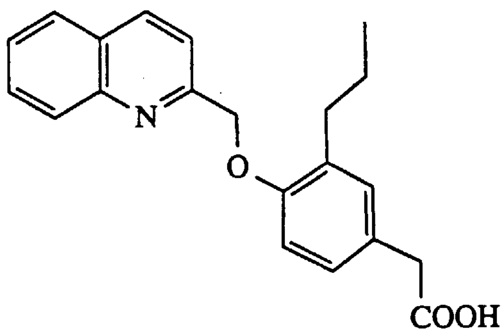

In Analogie zur Vorschrift des Beispiels XV wird die Titelverbindung aus 1,5 g (4,3 mmol) der Verbindung aus Beispiel XXXIX und 10 ml (10 mmol) 1 n Natronlauge hergestellt.
Ausbeute: 1,4 g (97,2% der Theorie) farbl. Kristalle
Fp.: 135°C

Beispiel XLI

2-[3-Propyl-4-chinolin-2-yl-methoxy)phenyl]-acetylmethansulfonamid

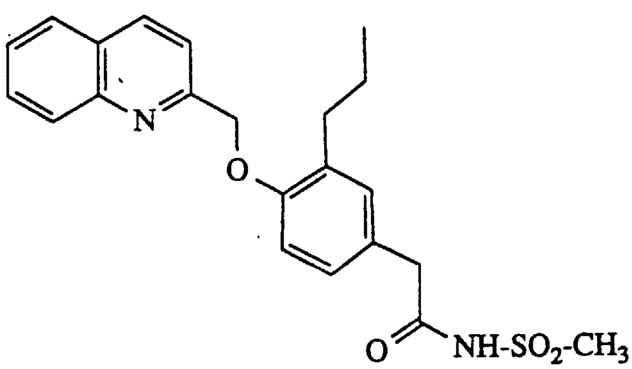

In Analogie zur Vorschrift des Beispiels XVI wird die Titelverbindung aus 1,7 g (5,1 mmol) der Verbindung aus Beispiel XL, 0,6 g (6 mmol) Methansulfonamid, 1,2 g (6 mmol) N-Ethyl-N'-dimethylaminocarbodiimid-hydrochlorid und 0,8 g (6 mmol) Dimethylaminopyridin hergestellt.
Ausbeute: 1,5 g (71,4% der Theorie) farbl. Kristalle
Fp.: 155°C (Zers.)

Beispiel XLII

2-[3-Acetyl-4-(chinolin-2-yl-methoxy)phenyl]essigsäuremethylester

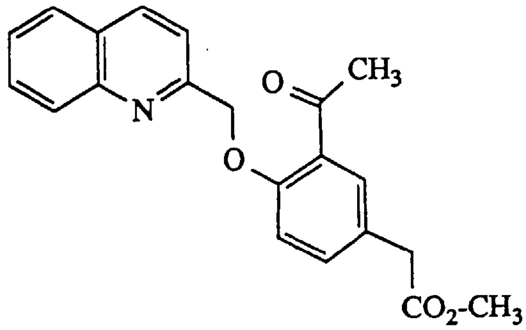

In Analogie zur Vorschrift des Beispiels XIV wird die Titelverbindung aus 2,9 g (0,014 mol) der Verbindung aus Beispiel VIII, 2,5 g (0,014 mol) 2-Chlormethylchinolin und 0,56 g (0,014 mol) Natriumhydroxid hergestellt.
Ausbeute: 2,1 g (43,0% der Theorie) farbl. Öl.

Beispiel XLIII

2-[3-Acetyl-4-(chinolin-2-yl-methoxy)phenyl]essigsäure

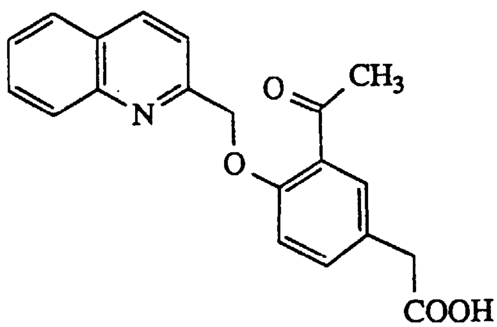

In Analogie zur Vorschrift des Beispiels XV wird die Titelverbindung aus 1 g (2,9 mmol) der Verbindung aus Beispiel XLII und 0,13 g (5,8 mmol) Lithiumhydroxid in 10 ml Wasser hergestellt.
Ausbeute: 0,7 g (72,1% der Theorie) farbl. Kristalle
Fp.: 119°C (Zers.)

Herstellungsbeispiele(allgemeine Formel I)

Beispiel 1

2-[3-Fluor4-(chinolin-2-methoxy)phenyl]-2-cyclopentylessigsäuremethylester

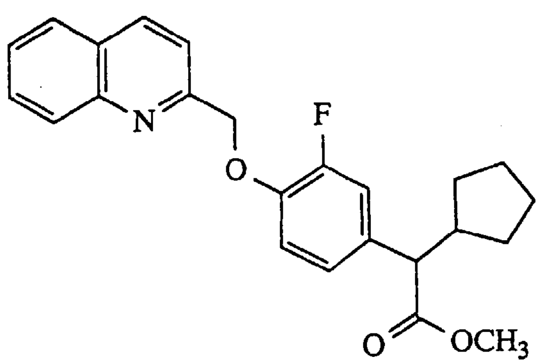

0,45 g (0,015 mol) 80%iges NaH werden unter Argon in DMF suspendiert, und dazu werden 5 g (0,015 mmol) der

Verbindung aus Beispiel XIV in 80 ml DMF zugegeben. Nach Beendigung der Wasserstoffentwicklung wird noch 1 h nachgerührt. Dann tropft man innerhalb 1 h 2,4 g = 1,61 ml (0,015 mol) Cyclopentylbromid in 100 ml DMF zu und läßt über Nacht weiterreagieren. Man dampft das Lösemittel im Vakuum zur Trockene ein, nimmt in Dichlormethan auf, schüttelt mit verdünnter Salzsäure und $NaHCO_3$-Lösung aus, trocknet, engt auf ein kleines Volumen ein und trennt das Gemisch säulenchromatographisch (Kieselgel 60, Laufmittel: Toluol/Essigester = 9/1).
Ausbeute: 3,5 g (59,3% der Theorie) farbl. Kristalle
Fp.: 75°C

Beispiel 2

2-[3-Methoxy-4-(chinolin-2-yl-methoxy)phenyl]-2-cyclooctylessigsäuremethylester

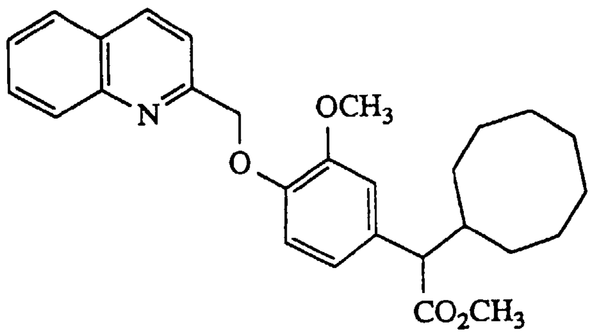

7,68 g (23 mmol) der Verbindung aus Beispiel XXIII und 4,4 g (23 mmol) Cyclooctylbromid werden in 100 ml Dimethylformamid gelöst. Dazu läßt man bei 0 - 10°C unter Rühren 3,36 g (30 mmol) Kaliumtertiärbutylat, gelöst in 30 ml DMF, zutropfen. Man läßt noch zwei Stunden bei Raumtemperatur nachrühren und versetzt dann mit 30 ml 1 n Salzsäure. Anschließend dampft man das Lösemittel im Vakuum ab, nimmt den Rückstand in 200 ml Dichlormethan auf und wäscht zweimal mit 100 ml Wasser. Nach Trocknen der Dichlormethanlösung mit Natriumsulfat engt man im Vakuum auf ein kleines Volumen ein und trennt den Rest säulenchromatographisch (Kieselgel 60, Laufmittel: Toluol/ Essigester = 4/1).
Ausbeute: 5,8 g (56,4% der Theorie) gelbes Öl

In Analogie zu den Vorschriften der Beispiele 1 und 2 werden die in Tabelle 1 aufgeführten Verbindungen hergestellt:

Tabelle 1

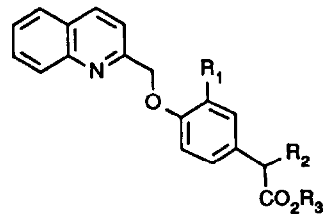

| Bsp.-Nr. | $R^1$ | $R^2$ | $R^3$ | F (°C) | Ausbeute (% d. Th.) |
|---|---|---|---|---|---|
| 3 | F | 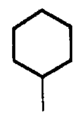 | $CH_3$ | 95 | 40,9 |
| 4 | F | 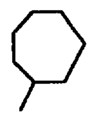 | $CH_3$ | 85 | 53,4 |
| 5 | Cl | 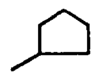 | $CH_3$ | Öl | 43,4 |
| 6 | Cl | 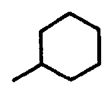 | $CH_3$ | Öl | 30,8 |
| 7 | Cl | 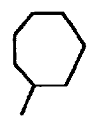 | $CH_3$ | Öl | 67 |
| 8 | Br | 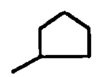 | $CH_3$ | 108-110 | 28,8 |

**Fortsetzung Tabelle 1**

| Bsp.-Nr. | $R^1$ | $R^2$ | $R^3$ | F (°C) | Ausbeute (% d. Th.) |
|---|---|---|---|---|---|
| 9 | Br | 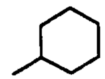 | $CH_3$ | | 19,2 |
| 10 | Br | 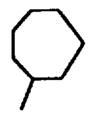 | $CH_3$ | Öl | 48 |
| 11 | $OCH_3$ | 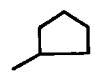 | $CH_3$ | Öl | 23,3 |
| 12 | $OCH_3$ | 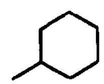 | $CH_3$ | Öl | 43,5 |
| 13 | $OCH_3$ | 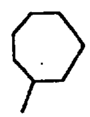 | $CH_3$ | Öl | 39,6 |
| 14 | 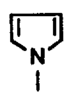 | 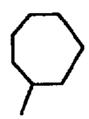 | $CH_3$ | Öl | 82,2 |

<u>Fortsetzung Tabelle 1</u>

| Bsp.-Nr. | $R^1$ | $R^2$ | $R^3$ | F (°C) | Ausbeute (% d. Th.) |
|---|---|---|---|---|---|
| 15 | 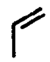 | 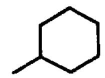 | $CH_3$ | Öl | 28,9 |
| 16 | 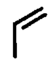 | 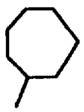 | $CH_3$ | Öl | 60,8 |
| 17 | 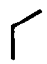 | 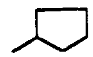 | $CH_3$ | Öl | 30,5 |
| 18 | 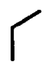 | 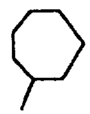 | $CH_3$ | Öl | 61,8 |
| 19 | 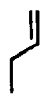 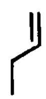 1 : 1 | 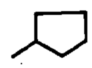 | $CH_3$ | Öl | 50,4 |
| 20 | 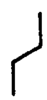 | 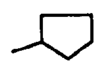 | $CH_3$ | Öl | 75,6 |

Fortsetzung Tabelle 1

| Bsp.-Nr. | $R^1$ | $R^2$ | $R^3$ | F (°C) | Ausbeute (% d. Th.) |
|---|---|---|---|---|---|
| 21 | 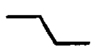 | 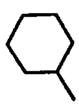 | $CH_3$ | Öl | 35,0 |
| 22 | 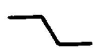 | 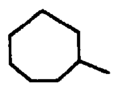 | $CH_3$ | Öl | 86,7 |
| 23 | 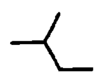 | 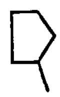 | $CH_3$ | Öl | 81,7 |
| 24 | $-CO-CH_3$ | 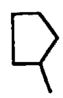 | $CH_3$ | 127 | 71,9 |
| 25 | $N_3$ | 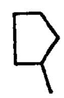 | $CH_3$ | 102-104 | 77 |
| 26 | $-NO_2$ | 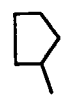 | $CH_3$ | 91-93 | 71,4 |

Beispiel 27

2-[3-Fluor-4-(chinolin-2-yl-methoxy)phenyl]-2-cyclopentylessigsäure

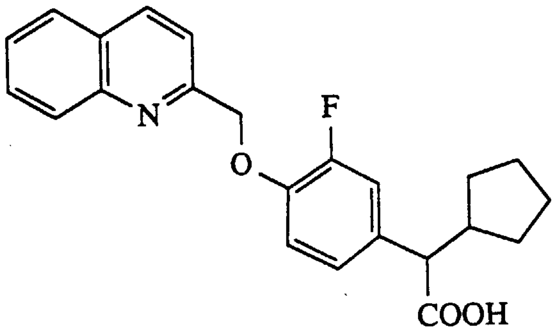

In Analogie zur Vorschrift des Beispiels XV wird die Titelverbindung aus 2,5 g (0,00635 mol) der Verbindung aus Beispiel 1 und 9,35 ml 1 molarer Natronlauge (0,00935 mol) hergestellt.
Ausbeute: 1,9 g (78,8% der Theorie) farbl. Kristalle
Fp.: 143 - 145°C

Die in Tabelle 2 aufgeführten Verbindungen wurden in Analogie zur Vorschrift des Beispiels 27 hergestellt:

**Tabelle 2**

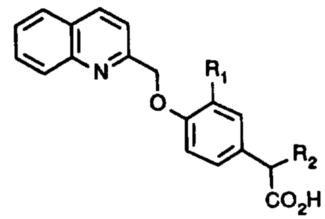

| Bsp.-Nr. | $R^1$ | $R^2$ | F (°C) | Ausbeute (% d. Th.) |
|---|---|---|---|---|
| 28 | F | 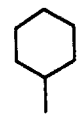 | 188-190 | 62,1 |
| 29 | F | 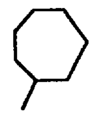 | 145 | 90,1 |
| 30 | Cl | 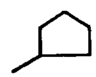 | 195 | 94,4 |
| 31 | Cl | 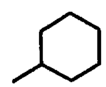 | 175 | 97,7 |
| 32 | Cl | 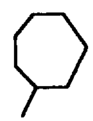 | 158-160 | 95,5 |
| 33 | Br | 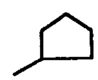 | 108-110 | 92,1 |

<u>Fortsetzung Tabelle 2</u>

| Bsp.-Nr. | $R^1$ | $R^2$ | F (°C) | Ausbeute (% d. Th.) |
|---|---|---|---|---|
| 34 | Br | 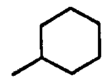 | 165 | 58,9 |
| 35 | Br | 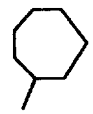 | 177 | 74,9 |
| 36 | $OCH_3$ | 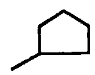 | 197 | 66,6 |
| 37 | $OCH_3$ | 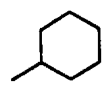 | 197-199 | 72,4 |
| 38 | $OCH_3$ | 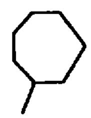 | 174 | 80,0 |
| 39 | $OCH_3$ | 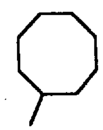 | 167 | 94,2 |

Fortsetzung Tabelle 2

| Bsp.-Nr. | $R^1$ | $R^2$ | F (°C) | Ausbeute (% d. Th.) |
|---|---|---|---|---|
| 40 | 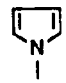 | 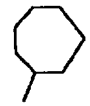 | 165 | 96,3 |
| 41 | 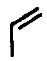 | 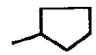 | 170 | 99,5 |
| 42 | 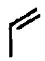 | 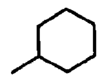 | 133 | 98,6 |
| 43 | 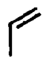 | 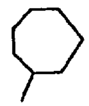 | 153 | 90,3 |
| 44 | 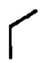 | 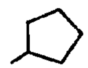 | 136 | 89,9 |
| 45 | 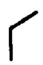 | 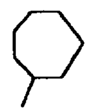 | 161 | 95,4 |
| 46 | 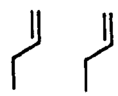 1 : 1 | 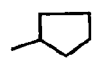 | 133 | 97,2 |

Fortsetzung Tabelle 2

| Bsp.-Nr. | $R^1$ | $R^2$ | F (°C) | Ausbeute (% d. Th.) |
|---|---|---|---|---|
| 47 | 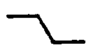 | 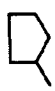 | 136 | 92,2 |
| 48 | 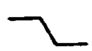 | 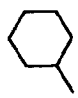 | 159 | 94,1 |
| 49 | 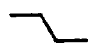 | 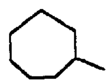 | 129 | 97,1 |
| 50 | 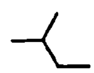 | 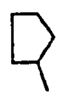 | 120 | 85,6 |
| 51 | $-CO-CH_3$ | 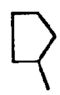 | 210 | 85,7 |
| 52 | $N_3$ | 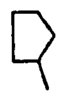 | 76-79 | 65 |

Beispiel 53

{2-[3-Fluor-(4-chinolin-2-yl-methoxy)phenyl]-2-cycloheptylacetyl}-methansulfonamid

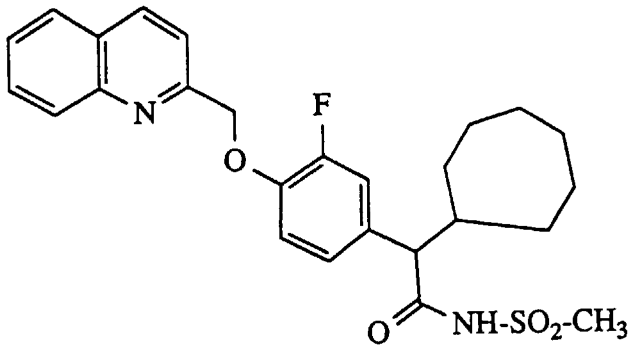

In Analogie zur Vorschrift des Beispiels XVI wird die Titelverbindung aus 1,7 g (0,0042 mol) der Verbindung aus Beispiel 29, 0,4 g (0,042 mol) getrocknetem Methansulfonamid, 0,81 g (0,0042 mol) N-Ethyl-N'-dimethylaminopropylcarbodiimid-hydrochlorid und 0,51 g (0,0042 mol) Dimethylaminopyridin hergestellt.

In Analogie zur Vorschrift des Beispiels 53 werden die in Tabelle 3 aufgeführten Verbindungen hergestellt:

Tabelle 3

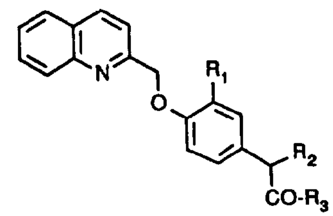

| Bsp.-Nr. | $R^1$ | $R^2$ | $R^3$ | F (°C) | Ausbeute (% d. Th.) |
|---|---|---|---|---|---|
| 54 | F | 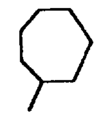 | $-NH-SO_2-CH_2-C_6H_5$ | 105 | 83,3 |
| 55 | Cl | 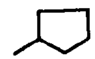 | $-NH-SO_2-CH_2-C_6H_5$ | 184 | 66,4 |
| 56 | Cl | 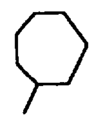 | $-NH-SO_2-CH_3$ | 80 | 66 |
| 57 | Cl | 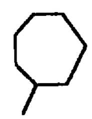 | $-NH-SO_2-CH_2-C_6H_5$ | 188 | 77 |
| 58 | Br | 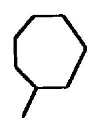 | $-NH-SO_2-CH_3$ | 189 | 63,2 |
| 59 | $OCH_3$ | 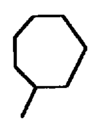 | $-NH-SO_2-CH_3$ | 130 | 79,7 |

Fortsetzung Tabelle 3

| Bsp.-Nr. | $R^1$ | $R^2$ | $R^3$ | F (°C) | Ausbeute (% d. Th.) |
|---|---|---|---|---|---|
| 60 | $OCH_3$ | 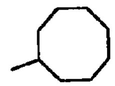 | $-NH-SO_2-CH_3$ | 123 | 59,7 |
| 61 | $OCH_3$ | 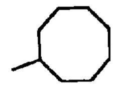 | $-NH-SO_2-CH(CH_3)_2$ | 103 | 80,8 |
| 62 | 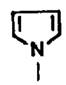 | 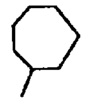 | $-NH-SO_2-CH_3$ | 196-198 | 77 |
| 63 | 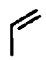 | 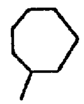 | $-NH-SO_2-CH_3$ | 157 | 56,4 |
| 64 | 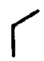 | 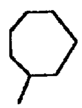 | $-NH-SO_2-CH_3$ | 143 | 74,7 |
| 65 | 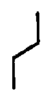 | 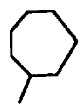 | $-NH-SO_2-CH_3$ | 147 | 70.7 |
| 66 | 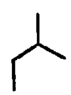 | 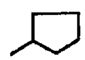 | $-NH-SO_2-CH_3$ | 97 | 79,5 |
| 67 | $N_3$ | 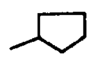 | $-NH-SO_2-(C_6H_5)-p-J$ | 63-67 | 11 |

Die in Tabelle 4 aufgeführten Verbindungen wurden in Analogie zur Vorschrift des Beispiels 27 hergestellt:

Tabelle 4

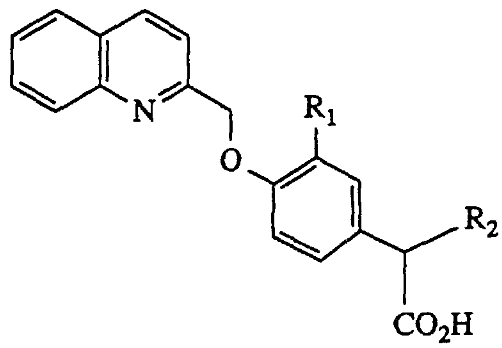

| Bsp.-Nr. | $R^1$ | $R^2$ | F °C | Ausbeute (% d.Th.) |
|---|---|---|---|---|
| 68 | 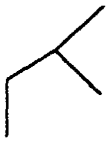 | 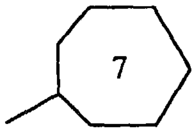 | 112 | 92,7 |
| 69 | 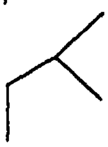 | 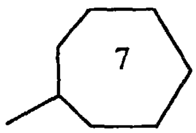 | (-)-Enantiomer | |
| 70 | 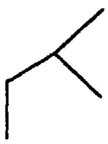 | 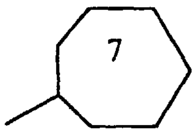 | (+)-Enantiomer | |
| 71 | 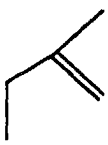 | 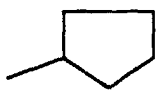 | 123 | 75,1 |
| 72 | 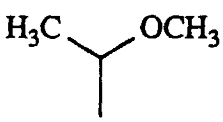 | 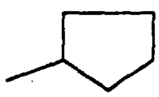 | 115 (Zersetzung) | |
| 73 | 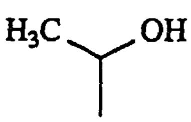 | 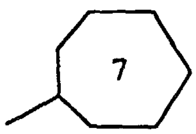 | Schaum | 47,9 |

Die in Tabelle 5 aufgeführten Verbindungen wurden in Analogie zur Vorschrift des Beispiels 53 hergestellt:

Tabelle 5:

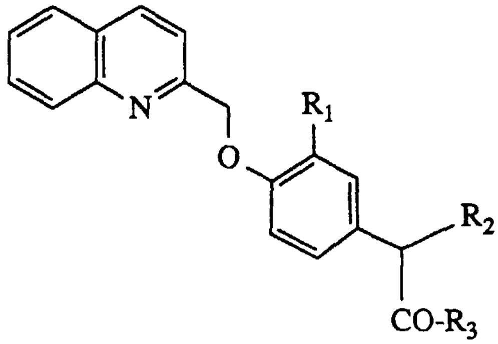

| Bsp.-Nr. | $R^1$ | $R^2$ | $R^3$ | F °C | Ausbeute (% d.Th.) |
|---|---|---|---|---|---|
| 74 | 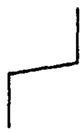 | 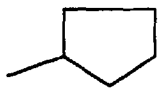 | $-NH-SO_2-CH_3$ | 140 | 58,0 |
| 75 | 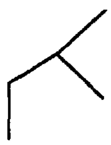 | 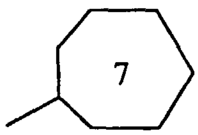 | $-NH-SO_2-CH_3$ | amorph | 27 |
| 76 | 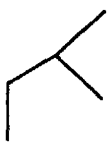 | 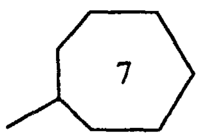 | $-NH-SO_2$ 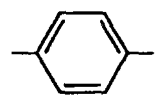 $CH_3$ | 189 | 89,2 |
| 77 | 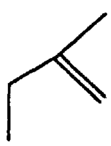 | 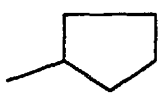 | $-NH-SO_2$ 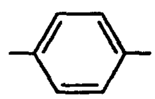 $CH_3$ | amorph | 95 |
| 78 | 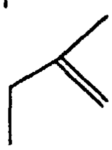 | 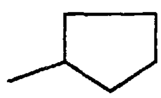 | $-NH-SO_2-CH_3$ | amorph | 92,3 |

Die in Tabelle 6 aufgeführten Verbindungen wurden in Analogie zur Vorschrift des Beispiels 2 hergestellt:

Tabelle 6:

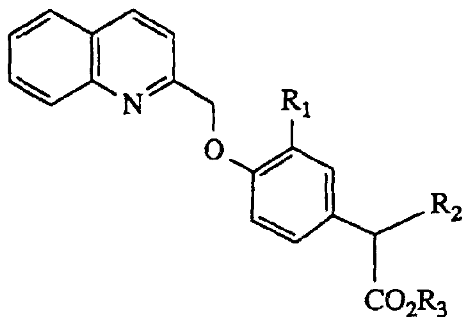

| Bsp.-Nr. | $R^1$ | $R^2$ | $R^3$ | F °C | Ausbeute (% d.Th.) |
|---|---|---|---|---|---|
| 79 | 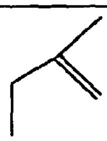 | 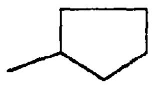 | $-CH_3$ | Öl | 95,2 |

## Patentansprüche

**Patentansprüche für folgende Vertragsstaaten : AT, BE, CH, DE, DK, FR, GB, GR, IT, LI, LU, NL, PT, SE**

**1.** 2-substituierte Chinoline der allgemeinen Formel

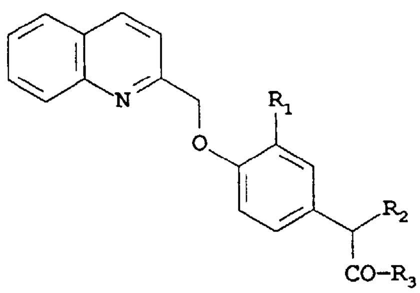

worin

$R^1$ für Fluor, Chlor, Brom, Iod, Cyano, Nitro, Azido, Trifluormethyl oder Trifluormethoxy steht, oder für geradkettiges oder verzweigtes Alkoxy oder Acyl mit jeweils bis zu 6 Kohlenstoffatomen steht, oder für geradkettiges oder verzweigtes Alkyl mit bis zu 6 Kohlenstoffatomen steht, das gegebenenfalls durch Hydroxy oder Alkoxy mit bis zu 4 Kohlenstoffatomen substituiert ist, oder für geradkettiges oder verzweigtes Alkenyl mit bis zu 4 Kohlenstoffatomen steht, oder
für eine Gruppe der Formel $-NR^4R^5$ steht,
worin

$R^4$ und $R^5$ gleich oder verschieden sind und Wasserstoff oder geradkettiges oder verzweigtes Alkyl mit bis zu 4 Kohlenstoffatomen bedeuten,

oder für Pyrryl, Pyridyl, Furyl oder Phenyl steht,

$R^2$ für Cycloalkyl mit 3 bis 12 Kohlenstoffatomen steht,

$R^3$ für einen Rest der Formel $-OR^6$ oder $-NR^7-SO_2-R^8$ steht, worin

$R^6$ Wasserstoff oder geradkettiges oder verzweigtes Alkyl mit bis zu 6 Kohlenstoffatomen bedeutet,

$R^7$ Wasserstoff oder geradkettiges oder verzweigtes Alkyl mit bis zu 4 Kohlenstoffatomen bedeutet,

$R^8$ Phenyl bedeutet, das gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl doer druch geradkettiges oder verzweigtes Alkyl oder Alkoxy mit jeweils bis zu 4 Kohlenstoffatomen substituiert sein kann

und deren physiologisch unbedenklichen Salze.

**2.** 2-substituierte Chinoline nach Anspruch 1, worin

$R^1$ für Fluor, Chlor, Brom, Nitro, Azido oder Trifluormethoxy steht, oder für geradkettiges oder verzweigtes Alkoxy oder Acyl mit jeweils bis zu 4 Kohlenstoffatomen steht, oder für geradkettiges oder verzweigtes Alkyl mit bis zu 4 Kohlenstoffatomen steht, das gegebenenfalls durch Hydroxy oder durch Methoxy substituiert ist, oder für geradkettiges oder verzweigtes Alkenyl mit bis zu 4 Kohlenstoffatomen steht oder für eine Gruppe der Formel $-NR^4R^5$ steht, worin

$R^4$ und $R^5$ gleich oder verschieden sind und Wasserstoff oder geradkettiges oder verzweigtes Alkyl mit bis zu 3 Kohlenstoffatomen bedeuten,

oder für Pyrryl, Furyl oder Phenyl steht,

$R^2$ für Cyclobutyl, Cyclopentyl, Cyclohexyl, Cycloheptyl oder Cyclooctyl steht,

$R^3$ für einen Rest der Formel $-OR^6$ oder $-NR^7-SO_2-R^8$ steht, worin

$R^6$ Wasserstoff oder geradkettiges oder verzweigtes Alkyl mit bis zu 4 Kohlenstoffatomen bedeutet,

$R^7$ Wasserstoff, Methyl oder Ethyl bedeutet,

$R^8$ Phenyl bedeutet, das gegebenenfalls durch Methyl, Fluor, Chlor, Brom, Iod, Methoxy oder Triluormethyl substituiert ist, oder geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann

und deren physiologisch unbedenklichen Salze.

**3.** 2-substituierte Chinoline nach Anspruch 1 bis 2 zur Behandlung von Krankheiten.

**4.** Verfahren zur Herstellung von 2-substituierten Chinolinen nach Anspruch 1 bis 2 dadurch gekennzeichnet, daß

man

[A] im Fall, daß $R^3$ für die Gruppe $-OR^6$ steht
[$A_1$] entweder Verbindungen der allgemeinen Formel (IIa)

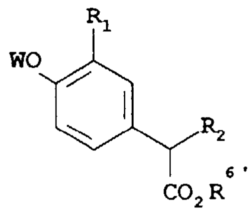

(IIa)

in welcher
$R^1$ und $R^2$ die oben angegebene Bedeutung haben,
W für eine Hydroxyschutzgruppe wie Benzyl oder tert.Butyl steht
und
$R^{6'}$ die oben angebene Bedeutung von $R^6$ hat aber nicht für Wasserstoff steht,
nach Abspaltung der Schutzgruppe mit 2-Halogenmethylchinolinen der allgemeinen Formel (III)

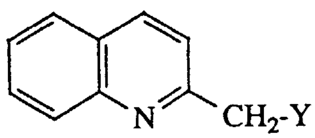

(III),

in welcher
Y für Halogen, insbesondere für Chlor oder Brom steht,
in inerten Lösemitteln durch Veretherung in die Verbindungen der allgemeinen Formel (IVa)

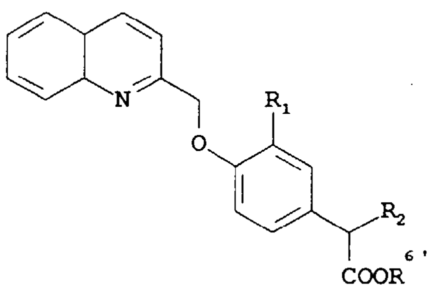

(IVa)

in welchen
$R^1$, $R^2$ und $R^{6'}$ die oben angegebene Bedeutung haben,
überführt und diese im Fall der Ester ($R^6 \neq H$) anschließend verseift,
oder
[$A_2$] Verbindungen der allgemeinen Formel (IIb)

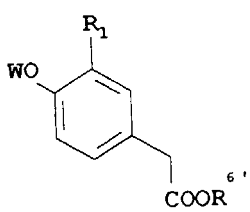

(IIb)

in welcher $R^1$ und $R^{6'}$ die oben angegebene Bedeutung haben,
nach Abspaltung der Schutzgruppe zunächst mit 2 Halogenmethylchinolinen der allgemeinen Formel (III) in inerten Lösemitteln durch Veretherung in Verbindungen der allgemeinen Formel (IVb)

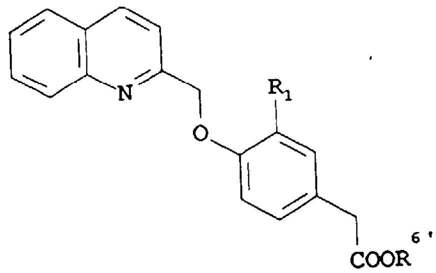

(IVb)

in welcher

$R^1$ und $R^{6'}$ die oben angegebene Bedeutung haben,

überführt,

und diese anschließend mit Verbindungen der allgemeinen Formel (V)

$$R^2\text{-}Z \qquad (V),$$

in welcher

$R^2$ die oben angegebene Bedeutung hat

und

Z für Chlor, Brom oder Iod steht,

in inerten Lösemitteln alkyliert und im Fall der Ester ($R^6 \neq H$) die Ester verseift,

oder

[B] im Fall, daß $R^3$ für die Gruppe $-NR^7-SO_2R^8$ steht,

Verbindungen der allgemeinen Formel (IVc)

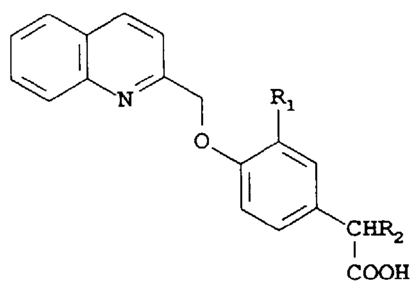

(IVc)

in welcher

$R^1$ und $R^2$ die oben angegebene Bedeutung haben,

in inerten Lösemitteln, gegebenenfalls in Anwesenheit einer Base mit Sulfonamiden der allgemeinen Formel (VI)

$$HNR^7\text{-}SO_2R^8 \qquad (VI),$$

in welcher

$R^7$ und $R^8$ die oben angegebene Bedeutung haben,

amidiert,

und

[C] im Fall der Enantiomeren die entsprechenden enantiomerenreinen Säuren (IVc) nach üblicher Methode trennt und nach den oben aufgeführten Verfahren weiter umsetzt.

5. Arzneimittel enthaltend mindestens ein 2-substituiertes Chinolin nach Anspruch 1 bis 2.

6. Verfahren zur Herstellung eines Arzneimittels nach Anspruch 5, dadurch gekennzeichnet, daß man die 2-substituierten Chinoline gegebenenfalls mit Hilfe von üblichen Hilfs- und Trägerstoffen in eine geeignete Applikationsform bringt.

7. Verwendung der 2-substituierten Chinoline nach Anspruch 1 zur Herstellung von Arzneimitteln.

**8.** Verwendung nach Anspruch 7 zur Herstellung von Lipoxygenasehemmern.

**Patentanspruch für folgenden Vertragsstaat : ES**

**1.** Verfahren zur Herstellung von 2-substituierten Chinolinen der Formel

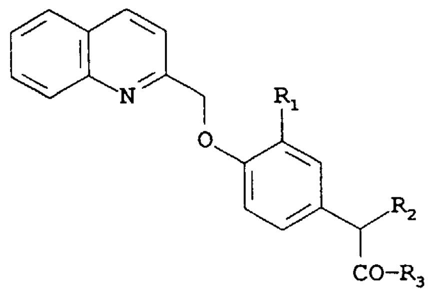

worin

$R^1$ für Fluor, Chlor, Brom, Iod, Cyano, Nitro, Azido, Trifluormethyl oder Trifluormethoxy steht, oder für geradkettiges oder verzweigtes Alkoxy oder Acyl mit jeweils bis zu 6 Kohlenstoffatomen steht, oder für geradkettiges oder verzweigtes Alkyl mit bis zu 6 Kohlenstoffatomen steht, das gegebenenfalls durch Hydroxy oder Alkoxy mit bis zu 4 Kohlenstoffatomen substituiert ist, oder für geradkettiges oder verzweigtes Alkenyl mit bis zu 4 Kohlenstoffatomen steht, oder für eine Gruppe der Formel $-NR^4R^5$ steht, worin

$R^4$ und $R^5$ gleich oder verschieden sind und Wasserstoff oder geradkettiges oder verzweigtes Alkyl mit bis zu 4 Kohlenstoffatomen bedeuten,

oder für Pyrryl, Pyridyl, Furyl oder Phenyl steht,

$R^2$ für Cycloalkyl mit 3 bis 12 Kohlenstoffatomen steht,

$R^3$ für einen Rest der Formel $-OR^6$ oder $-NR^7-SO_2-R^8$ steht, worin

$R^6$ Wasserstoff oder geradkettiges oder verzweigtes Alkyl mit bis zu 6 Kohlenstoffatomen bedeutet,

$R^7$ Wasserstoff oder geradkettiges oder verzweigtes Alkyl mit bis zu 4 Kohlenstoffatomen bedeutet,

$R^8$ Phenyl bedeutet, das gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl doer druch geradkettiges oder verzweigtes Alkyl oder Alkoxy mit jeweils bis zu 4 Kohlenstoffatomen substituiert sein kann

dadurch gekennzeichnet, daß man

[A] im Fall, daß $R^3$ für die Gruppe $-OR^6$ steht
[$A_1$] entweder Verbindungen der allgemeinen Formel (IIa)

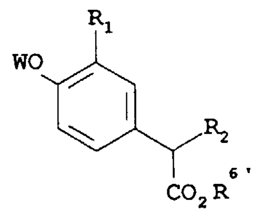

(IIa)

in welcher
$R^1$ und $R^2$ die oben angegebene Bedeutung haben,
W für eine Hydroxyschutzgruppe wie Benzyl oder tert.Butyl steht
und
$R^{6'}$ die oben angebene Bedeutung von $R^6$ hat aber nicht für Wasserstoff steht,
nach Abspaltung der Schutzgruppe mit 2-Halogenmethylchinolinen der allgemeinen Formel (III)

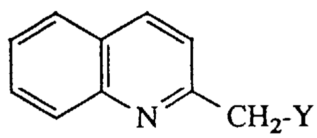

(III),

in welcher
und
Y für Halogen, insbesondere für Chlor oder Brom steht,
in inerten Lösemitteln durch Veretherung in die Verbindungen der allgemeinen Formel (IVa)

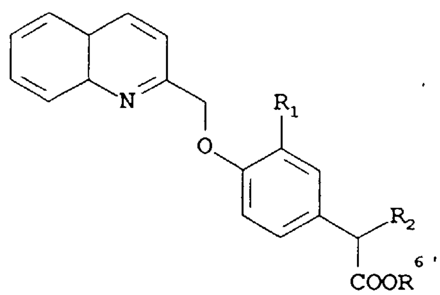

(IVa)

in welchen
$R^1$, $R^2$ und $R^{6'}$ die oben angegebene Bedeutung haben,
überführt und diese im Fall der Ester ($R^6 \neq H$) anschließend verseift,
oder
[$A_2$] Verbindungen der allgemeinen Formel (IIb)

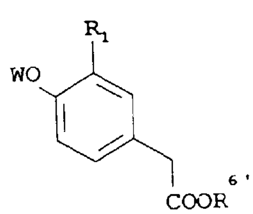

(IIb)

in welcher $R^1$ und $R^{6'}$ die oben angegebene Bedeutung haben,
nach Abspaltung der Schutzgruppe zunächst mit 2 Halogenmethylchinolinen der allgemeinen Formel (III) in inerten Lösemitteln durch Veretherung in Verbindungen der allgemeinen Formel (IVb)

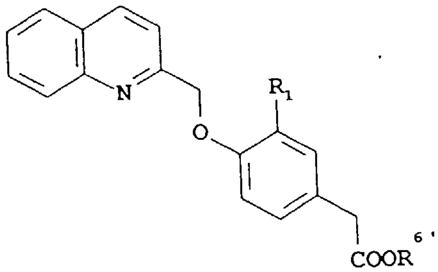

(IVb)

in welcher

$R^1$ und $R^{6'}$ die oben angegebene Bedeutung haben,

überführt,

und diese anschließend mit Verbindungen der allgemeinen Formel (V)

$$R^2\text{-}Z \quad (V),$$

in welcher

$R^2$ die oben angegebene Bedeutung hat

und

Z für Chlor, Brom oder Iod steht,

in inerten Lösemitteln alkyliert und im Fall der Ester ($R^6 \neq H$) die Ester verseift,

oder

[B] im Fall, daß $R^3$ für die Gruppe $-NR^7-SO_2R^8$ steht,

Verbindungen der allgemeinen Formel (IVc)

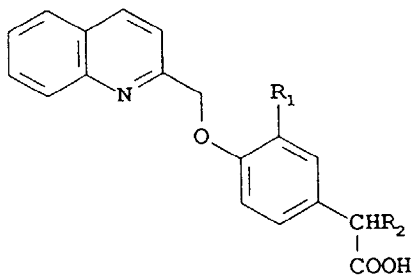

(IVc)

in welcher

$R^1$ und $R^2$ die oben angegebene Bedeutung haben,

in inerten Lösemitteln, gegebenenfalls in Anwesenheit einer Base mit Sulfonamiden der allgemeinen Formel (VI)

$$HNR^7\text{-}SO_2R^8 \quad (VI),$$

in welcher

$R^7$ und $R^8$ die oben angegebene Bedeutung haben,

amidiert,

und

[C] im Fall der Enantiomeren die entsprechenden enantiomerenreinen Säuren (IVc) nach üblicher Methode trennt und nach den oben aufgeführten Verfahren weiter umsetzt.

## Claims

**Claims for the following Contracting States : AT, BE, CH, DE, DK, FR, GB, GR, IT, LI, LU, NL, PT, SE**

**1.** 2-Substituted quinolines of the general formula,

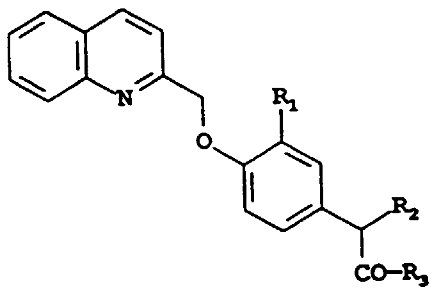

(I)

in which

$R^1$ represents fluorine, chlorine, bromine, iodine, cyano, nitro, azido, trifluoromethyl, trifluoromethoxy, or represents straight-chain or branched alkoxy or acyl each having up to 6 carbon atoms, or represents straight-chain or branched alkyl having up to 6 carbon atoms, which is optionally substituted by hydroxyl or alkoxy having up to 4 carbon atoms, or
represents straight-chain or branched alkenyl having up to 4 carbon atoms, or
represents a group of the formula $-NR^4R^5$,
in which

$R^4$ and $R^5$ are identical or different and denote hydrogen or straight-chain or branched alkyl having up to 4 carbon atoms,

or represent pyrryl, pyridyl, furyl or phenyl,

$R^2$ represents cycloalkyl having 3 to 12 carbon atoms,

$R^3$ represents a radical of the formula $-OR^6$ or
$-NR^7-SO_2-R^8$,
in which

$R^6$ denotes hydrogen or straight-chain or branched alkyl having up to 6 carbon atoms,

$R^7$ denotes hydrogen or straight-chain or branched alkyl having up to 4 carbon atoms,

$R^8$ denotes phenyl which is optionally substituted by fluorine, chlorine, bromine, iodine, cyano or by straight-chain or branched alkyl or alkoxy each having up to 6 carbon atoms, or
denotes straight-chain or branched alkyl having up to 6 carbon atoms, which is optionally substituted by phenyl which can in turn be substituted by fluorine, chlorine, bromine or trifluoromethyl or
by straight-chain or branched alkyl or
alkoxy each having up to 4 carbon atoms

and their physiologically acceptable salts.

**2.** 2-Substituted quinolines according to Claim 1, in which

$R^1$ represents fluorine, chlorine, bromine, nitro, azido or trifluoromethoxy, or
represents straight-chain or branched alkoxy or
acyl each having up to 4 carbon atoms, or
represents straight-chain or branched alkyl having up to 4 carbon atoms, which is optionally substituted by hydroxyl or methoxy, or represents straight-chain or branched alkenyl having up to 4 carbon atoms, or
represents a group of the formula $-NR^4R^5$, in which

$R^4$ and $R^5$ are identical or different and denote hydrogen or straight-chain or branched alkyl having

up to 3 carbon atoms,

or represents pyrryl, furyl or phenyl,

$R^2$ represents cyclobutyl, cyclopentyl, cyclohexyl, cycloheptyl or cyclooctyl,

$R^3$ represents a radical of the formula $-OR^6$ or $-NR^7-SO_2-R^8$, in which

$R^6$ denotes hydrogen or straight-chain or branched alkyl having up to 4 carbon atoms,

$R^7$ denotes hydrogen, methyl or ethyl,

$R^8$ denotes phenyl which is optionally substituted by methyl, fluorine, chlorine, bromine, iodine, methoxy or trifluoromethyl, or
denotes straight-chain or branched alkyl having up to 4 carbon atoms, which is optionally substituted by phenyl which can in turn be substituted by fluorine, chlorine, bromine, methyl or methoxy and their physiologically acceptable salts.

**3.** 2-substituted quinolines according to Claim 1 or 2 for the treatment of diseases.

**4.** Process for the preparation of 2-substituted quinolines according to Claim 1 or 2, characterised in that

[A] in the case where $R^3$ represents the group $-OR^6$
[$A_1$] either compounds of the general formula (IIa)

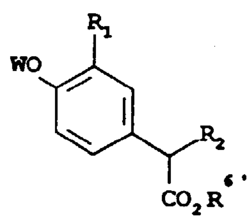

(IIa)

in which
$R^1$ and $R^2$ have the abovementioned meaning,
W represents a hydroxyl protective group such as benzyl or tert.-butyl,
and
$R^{6'}$ has the abovementioned meaning of $R^6$ but does not represent hydrogen,
are converted, after elimination of the protective group, by etherification with 2-halogenomethylquinolines of the general formula (III)

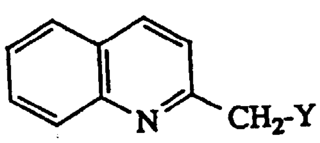

(III)

in which
Y represents halogen, in particular chlorine or bromine,
in inert solvents into the compounds of the general formula (IVa)

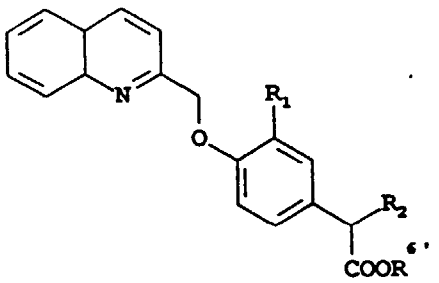

(IVa)

in which
$R^1$, $R^2$ and $R^{6'}$ have the abovementioned meaning,
and the latter in the case of the esters ($R^6 \neq H$) are then hydrolysed,
or
[$A_2$] compounds of the general formula (IIb)

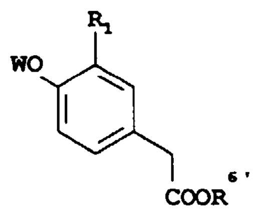

(IIb)

in which $R^1$ and $R^{6'}$ have the abovementioned meaning,
are converted, after elimination of the protective group, initially by etherification with 2-halogenomethylquinolines of the general formula (III) in inert solvents into compounds of the general formula (IVb)

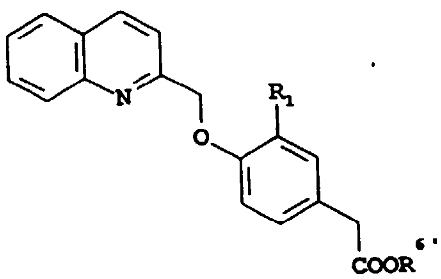

(IVb)

in which
$R^1$ and $R^{6'}$ have the abovementioned meaning,
and the latter are then alkylated with compounds of the general formula (V)

$$R^2\text{-}Z \qquad (V)$$

in which
$R^2$ has the abovementioned meaning
and
Z represents chlorine, bromine or iodine,
in inert solvents and in the case of the esters ($R^6 \neq H$) the esters are hydrolysed
or
[B] in the case where $R^3$ represents the group $-NR^7-SO_2R^8$,
compounds of the general formula (IVc)

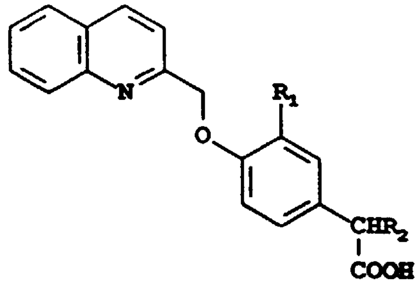

(IVc)

in which
$R^1$ and $R^2$ have the abovementioned meaning,
are amidated in inert solvents, if appropriate in the presence of a base, with sulphonamides of the general formula (VI)

$$HNR^7\text{-}SO_2R^8 \qquad (VI)$$

in which
$R^7$ and $R^8$ have the abovementioned meaning, and
[C] in the case of the enantiomers the corresponding enantiomerically pure acids (IVc) are separated by a customary method and reacted further by the abovementioned processes.

**5.** Medicaments containing at least one 2-substituted quinoline according to Claim 1 or 2.

**6.** Process for the production of a medicament according to Claim 5, characterised in that the 2-substituted quinolines are brought into a suitable administration form, if appropriate with the aid of customary auxiliaries and excipients.

**7.** Use of the 2-substituted quinolines according to Claim 1 for the production of medicaments.

**8.** Use according to Claim 7 for the production of lipoxygenase inhibitors.

**Claim for the following Contracting State : ES**

**1.** Process for the preparation of 2-substituted quinolines of the formula

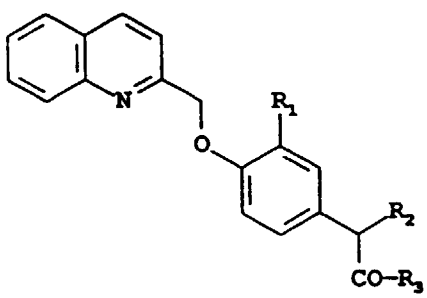

(I)

in which

$R^1$ represents fluorine, chlorine, bromine, iodine, cyano, nitro, azido, trifluoromethyl, trifluoromethoxy, or represents straight-chain or branched alkoxy or acyl each having up to 6 carbon atoms, or represents straight-chain or branched alkyl having up to 6 carbon atoms, which is optionally substituted by hydroxyl or alkoxy having up to 4 carbon atoms, or represents straight-chain or branched alkenyl having up to 4 carbon atoms, or represents a group of the formula $-NR^4R^5$, in which

$R^4$ and $R^5$ are identical or different and denote hydrogen or straight-chain or branched alkyl having up to

4 carbon atoms,

or represent pyrryl, pyridyl, furyl or phenyl,

$R^2$ represents cycloalkyl having 3 to 12 carbon atoms,

$R^3$ represents a radical of the formula $-OR^6$ or $-NR^7-SO_2-R^8$, in which

$R^6$ denotes hydrogen or straight-chain or branched alkyl having up to 6 carbon atoms,

$R^7$ denotes hydrogen or straight-chain or branched alkyl having up to 4 carbon atoms,

$R^8$ denotes phenyl which is optionally substituted by fluorine, chlorine, bromine, iodine, cyano or by straight-chain or branched alkyl or alkoxy each having up to 6 carbon atoms, or denotes straight-chain or branched alkyl having up to 6 carbon atoms, which is optionally substituted by phenyl which can in turn be substituted by fluorine, chlorine, bromine or trifluoromethyl or by straight-chain or branched alkyl or alkoxy each having up to 4 carbon atoms

characterised in that

[A] in the case where $R^3$ represents the group $-OR^6$
[$A_1$] either compounds of the general formula (IIa)

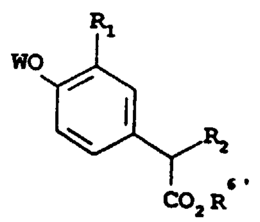

(IIa)

in which
$R^1$ and $R^2$ have the abovementioned meaning,
W represents a hydroxyl protective group such as benzyl or tert.-butyl,
and
$R^{6'}$ has the abovementioned meaning of $R^6$ but does not represent hydrogen,
are converted, after elimination of the protective group, by etherification with 2-halogenomethylquinolines of the general formula (III)

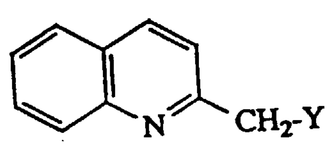

(III)

in which
Y represents halogen, in particular chlorine or bromine,
in inert solvents into the compounds of the general formula (IVa)

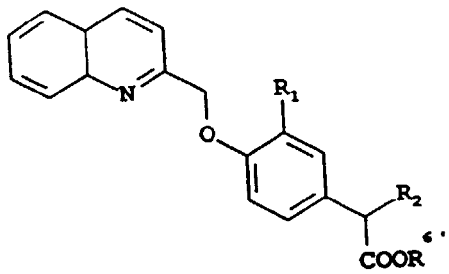

(IVa)

in which
$R^1$, $R^2$ and $R^{6'}$ have the abovementioned meaning,
and the latter in the case of the esters ($R^6 \neq H$) are then hydrolysed,
or
[$A_2$] compounds of the general formula (IIb)

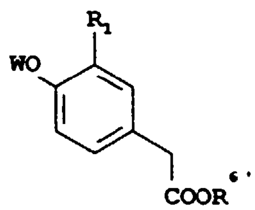

(IIb)

in which $R^1$ and $R^{6'}$ have the abovementioned meaning,
are converted, after elimination of the protective group, initially by etherification with 2-halogenomethylquinolines of the general formula (III) in inert solvents into compounds of the general formula (IVb)

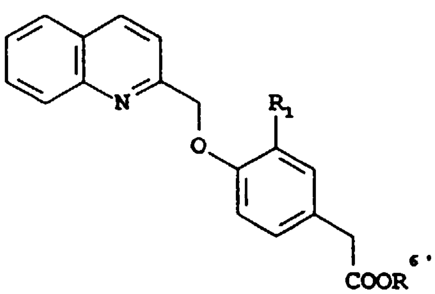

(IVb)

in which
$R^1$ and $R^{6'}$ have the abovementioned meaning,
and the latter are then alkylated with compounds of the general formula (V)

$$R^2\text{-}Z \qquad (V)$$

in which
$R^2$ has the abovementioned meaning
and
Z represents chlorine, bromine or iodine,
in inert solvents and in the case of the esters ($R^6 \neq H$) the esters are hydrolysed
or
[B] in the case where $R^3$ represents the group $-NR^7-SO_2R^8$,
compounds of the general formula (IVc)

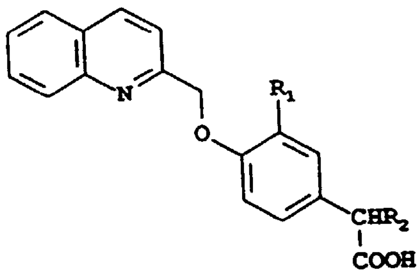

(IVc)

in which
$R^1$ and $R^2$ have the abovementioned meaning,
are amidated in inert solvents, if appropriate in the presence of a base, with sulphonamides of the general formula (VI)

$$HNR^7\text{-}SO_2R^8 \qquad (VI)$$

in which
$R^7$ and $R^8$ have the abovementioned meaning,
and
[C] in the case of the enantiomers the corresponding enantiomerically pure acids (IVc) are separated by a customary method and reacted further by the abovementioned processes.

## Revendications

### Revendications pour les Etats contractants suivants : AT, BE, CH, DE, DK, FR, GB, GR, IT, LI, LU, NL, PT, SE

**1.** Quinoléines 2-substituées répondant à la formule générale

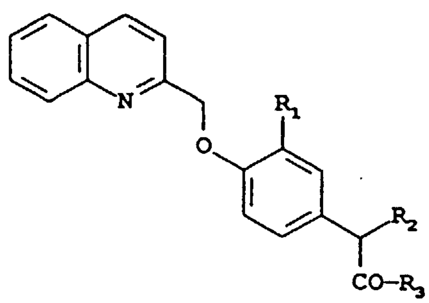

dans laquelle

$R^1$ représente un atome de fluor, un atome de chlore, un atome de brome, un atome d'iode, un groupe cyano, un groupe nitro, un groupe azido, un groupe trifluorométhyle ou un groupe trifluorométhoxy, ou
représente un groupe alcoxy ou un groupe acyle à chaîne droite ou ramifiée contenant respectivement jusqu'à 6 atomes de carbone, ou
représente un groupe alkyle à chaîne droite ou ramifiée contenant jusqu'à 6 atomes de carbone, qui porte éventuellement un ou plusieurs substituants identiques ou différents hydroxyle ou alcoxy contenant jusqu'à 4 atomes de carbone, ou
représente un groupe alcényle à chaîne droite ou ramifiée contenant jusqu'à 4 atomes de carbone, ou
représente un groupe de formule $-NR^4R^5$,
dans laquelle

$R^4$ et $R^5$ sont identiques ou différents et représentent un atome d'hydrogène ou un groupe alkyle à chaîne droite ou ramifiée contenant jusqu'à 4 atomes de carbone,

ou représente un groupe pyrryle, un groupe pyridyle, un groupe furyle ou un groupe phényle,

$R^2$ représente un groupe cycloalkyle contenant de 3 à 12 atomes de carbone,

$R^3$ représente un radical de formule $-OR^6$ ou $-NR^7-SO_2-R^8$ dans laquelle

$R^6$ représente un atome d'hydrogène ou un groupe alkyle à chaîne droite ou ramifiée contenant jusqu'à 6 atomes de carbone,

$R^7$ représente un atome d'hydrogène ou un groupe alkyle à chaîne droite ou ramifiée contenant jusqu'à 4 atomes de carbone,

$R^8$ représente un groupe phényle qui porte éventuellement un ou plusieurs substituants identiques ou différents fluoro, chloro, bromo, iodo, cyano ou encore un ou plusieurs substituants identiques ou différents alkyle ou alcoxy contenant respectivement jusqu'à 6 atomes de carbone, ou représente un groupe alkyle à chaîne droite ou ramifiée contenant jusqu'à 6 atomes de carbone, qui porte éventuellement un ou plusieurs substituants phényle qui pour leur part peuvent porter un ou plusieurs substituants identiques ou
différents fluoro, chloro, bromo, trifluorométhyle ou bien un ou plusieurs substituants identiques ou différents alkyle ou alcoxy à chaîne droite ou ramifiée contenant jusqu'à 4 atomes de carbone,

ainsi que leurs sels physiologiquement acceptables.

**2.** Quinoléines 2-substituées selon la revendication 1, dans lesquelles

$R^1$ représente un atome de fluor, un atome de chlore, un atome de brome, un groupe nitro, un groupe azido ou un groupe trifluorométhoxy, ou
représente un groupe alcoxy ou un groupe acyle à chaîne droite ou ramifiée contenant respectivement jusqu'à 4 atomes de carbone, ou
représente un groupe alkyle à chaîne droite ou ramifiée contenant jusqu'à 4 atomes de carbone, qui porte éventuellement un ou plusieurs substituants identiques ou différents hydroxyle ou méthoxy, ou
représente un groupe alcényle à chaîne droite ou ramifiée contenant jusqu'à 4 atomes de carbone, ou représente un groupe de formule $-NR^4R^5$,
dans laquelle

$R^4$ et $R^5$ sont identiques ou différents et représentent un atome d'hydrogène ou un groupe alkyle à chaîne droite ou ramifiée contenant jusqu'à 3 atomes de carbone,

ou représente un groupe pyrryle, un groupe furyle ou un groupe phényle,

$R^2$ représente un groupe cyclobutyle, un groupe cyclopentyle, un groupe cyclohexyle, un groupe cycloheptyle ou un groupe cyclooctyle,

$R^3$ représente un radical de formule $-OR^6$ ou $-NR^7-SO_2-R^8$ dans laquelle

$R^6$ représente un atome d'hydrogène ou un groupe alkyle à chaîne droite ou ramifiée contenant jusqu'à 4 atomes de carbone,

$R^7$ représente un atome d'hydrogène, un groupe méthyle ou un groupe éthyle,

$R^8$ représente un groupe phényle qui porte éventuellement un ou plusieurs substituants identiques ou différents méthyle, fluoro, chloro, bromo, iodo, méthoxy ou trifluorométhyle, ou
représente un groupe alkyle à chaîne droite ou ramifiée contenant jusqu'à 4 atomes de carbone, qui porte éventuellement un ou plusieurs substituants phényle qui pour leur part peuvent porter

un ou plusieurs substituants identiques ou différents fluoro, chloro, bromo, méthyle ou méthoxy,

ainsi que leurs sels physiologiquement acceptables.

**3.** Quinoléines 2-substituées selon les revendications 1 à 2 pour le traitement de maladies.

**4.** Procédé pour la préparation de quinoléines 2-substituées selon les revendications 1 à 2, caractérisé en ce que

[A] dans le cas où $R^3$ représente le groupe $-OR^6$, soit

[$A_1$] on transforme des composés répondant à la formule générale (IIa)

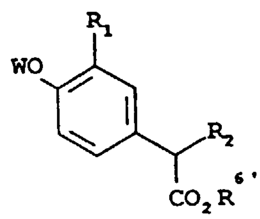

(IIa)

dans laquelle
$R^1$ et $R^2$ ont la signification indiquée ci-dessus,
W représente un groupe protecteur du groupe hydroxyle, tel qu'un groupe benzyle ou un groupe tert.-butyle,
et
$R^{6'}$ a la signification de $R^6$ indiquée ci-dessus, mais ne représente pas un atome d'hydrogène,
après élimination du groupe protecteur, avec des 2-halogénométhylquinoléines répondant à la formule générale (III)

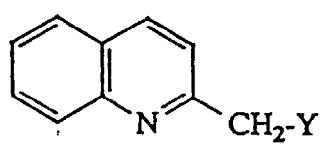

(III),

dans laquelle
Y représente un atome d'halogène, en particulier un atome de chlore ou un atome de brome,
dans des solvants inertes par éthérification, pour obtenir les composés répondant à la formule générale (IVa)

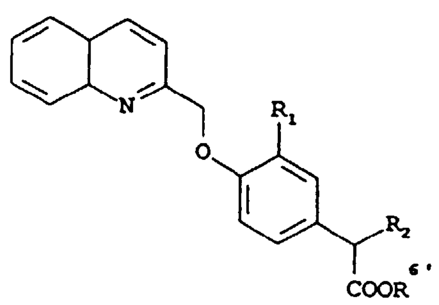

(IVa)

dans laquelle
$R^1$, $R^2$ et $R^{6'}$ ont la signification indiquée ci-dessus,
et on soumet ces derniers, dans le cas des esters ($R^6 \neq H$), à une saponification ultérieure, ou soit

[$A_2$] on transforme des composés répondant à la formule générale (IIb)

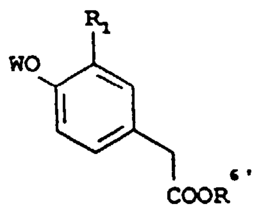

(IIb)

dans laquelle

$R^1$ et $R^{6'}$ ont la signification indiquée ci-dessus,

après élimination du groupe protecteur, d'abord avec des 2-halogénométhylquinoléines répondant à la formule générale (III) dans des solvants inertes par éthérification pour obtenir les composés répondant à la formule générale (IVb)

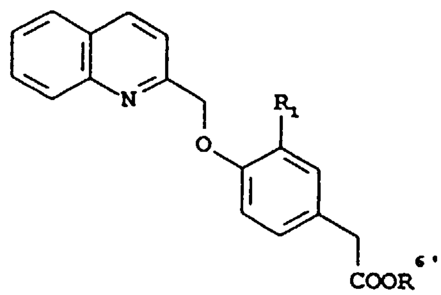

(IVb)

dans laquelle

$R^1$ et $R^{6'}$ ont la signification indiquée ci-dessus,

et ensuite on soumet ces derniers à une alkylation avec des composés répondant à la formule générale (V)

$$R^2\text{-}Z \qquad (V)$$

dans laquelle

$R^2$ a la signification indiquée ci-dessus

et

Z représente un atome de chlore, un atome de brome ou un atome d'iode,

dans des solvants inertes, et dans le cas des esters ($R^6 \neq H$), on soumet les esters à une saponification, ou bien

[B] dans le cas où $R^3$ représente le groupe $-NR^7-SO_2R^8$, on soumet à une amidation des composés répondant à la formule générale (IVc)

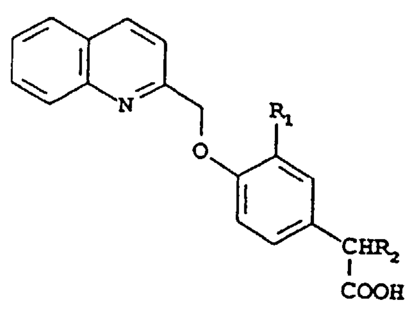

(IVc)

dans laquelle

$R^1$ et $R^2$ ont la signification indiquée ci-dessus,

dans des solvants inertes, éventuellement en présence d'une base, avec des sulfonamides répondant à la formule générale (VI)

$$HNR^7\text{-}SO_2R^8 \qquad (VI)$$

dans laquelle
$R^7$ et $R^8$ ont la signification indiquée ci-dessus, et

[C] dans le cas des énantiomères, on sépare les acides correspondants (IVc) en leurs formes énantiomères pures et on soumet celles-ci à une mise en réaction ultérieure conformément aux procédés indiqués ci-dessus.

**5.** Médicament contenant au moins une quinoléine 2-substituée selon les revendications 1 à 2.

**6.** Procédé pour la préparation d'un médicament selon la revendication 5, caractérisé en ce qu'on amène les quinoléines 2-substituées à une forme d'application appropriée éventuellement à l'aide de substances auxiliaires et de support habituelles.

**7.** Utilisation des quinoléines 2-substituées selon la revendication 1 pour la préparation de médicaments.

**8.** Utilisation selon la revendication 7 pour la préparation d'inhibiteurs de la lipoxygénase.

**Revendication pour l'Etat contractant suivant : ES**

**1.** Procédé pour la préparation de quinoléines 2-substituées de formule

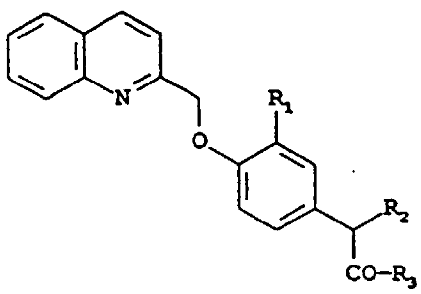

dans laquelle

$R^1$ représente un atome de fluor, un atome de chlore, un atome de brome, un atome d'iode, un groupe cyano, un groupe nitro, un groupe azido, un groupe trifluorométhyle ou un groupe trifluorométhoxy, ou
représente un groupe alcoxy ou un groupe acyle à chaîne droite ou ramifiée contenant respectivement jusqu'à 6 atomes de carbone, ou
représente un groupe alkyle à chaîne droite ou ramifiée contenant jusqu'à 6 atomes de carbone, qui porte éventuellement un ou plusieurs substituants identiques ou différents hydroxyle ou alcoxy contenant jusqu'à 4 atomes de carbone, ou
représente un groupe alcényle à chaîne droite ou ramifiée contenant jusqu'à 4 atomes de carbone, ou représente un groupe de formule $-NR^4R^5$,
dans laquelle

$R^4$ et $R^5$ sont identiques ou différents et représentent un atome d'hydrogène ou un groupe alkyle à chaîne droite ou ramifiée contenant jusqu'à 4 atomes de carbone,

ou représente un groupe pyrryle, un groupe pyridyle, un groupe furyle ou un groupe phényle,

$R^2$ représente un groupe cycloalkyle contenant de 3 à 12 atomes de carbone, '

$R^3$ représente un radical de formule $-OR^6$ ou $-NR^7\text{-}SO_2\text{-}R^8$
dans laquelle

$R^6$ représente un atome d'hydrogène ou un groupe alkyle à chaîne droite ou ramifiée contenant jusqu'à 6 atomes de carbone,

$R^7$ représente un atome d'hydrogène ou un groupe alkyle à chaîne droite ou ramifiée contenant jusqu'à 4 atomes de carbone,

$R^8$ représente un groupe phényle qui porte éventuellement un ou plusieurs substituants identiques ou différents fluoro, chloro, bromo, iodo, cyano ou encore un ou plusieurs substituants identiques ou différents alkyle ou alcoxy contenant respectivement jusqu'à 6 atomes de carbone, ou représente un groupe alkyle à chaîne droite ou ramifiée contenant jusqu'à 6 atomes de carbone, qui porte éventuellement un ou plusieurs substituants phényle qui pour leur part peuvent porter un ou plusieurs substituants identiques ou différents fluoro, chloro, bromo, trifluorométhyle ou bien un ou plusieurs substituants identiques ou différents alkyle ou alcoxy à chaîne droite ou ramifiée contenant jusqu'à 4 atomes de carbone,

caractérisé en ce que

[A] dans le cas où $R^3$ représente le groupe $-OR^6$, soit

[$A_1$] on transforme des composés répondant à la formule générale (IIa)

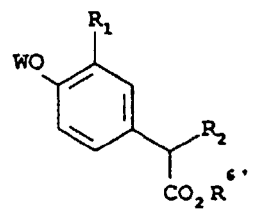

(IIa)

dans laquelle

$R^1$ et $R^2$ ont la signification indiquée ci-dessus,

W représente un groupe protecteur du groupe hydroxyle, tel qu'un groupe benzyle ou un groupe tert.-butyle,

et

$R^{6'}$ a la signification de $R^6$ indiquée ci-dessus, mais ne représente pas un atome d'hydrogène,

après élimination du groupe protecteur, avec des 2-halogénométhylquinoléines répondant à la formule générale (III)

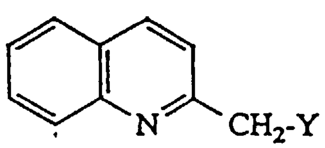

(III),

dans laquelle

Y représente un atome d'halogène, en particulier un atome de chlore ou un atome de brome,

dans des solvants inertes par éthérification, pour obtenir les composés répondant à la formule générale (IVa)

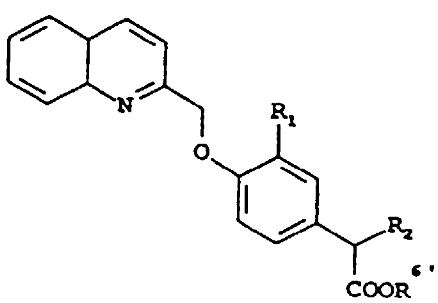

(IVa)

dans laquelle

$R^1$, $R^2$ et $R^{6'}$ ont la signification indiquée ci-dessus,

et on soumet ces derniers, dans le cas des esters ($R^6 \neq H$), à une saponification ultérieure, ou soit

[$A_2$] on transforme des composés répondant à la formule générale (IIb)

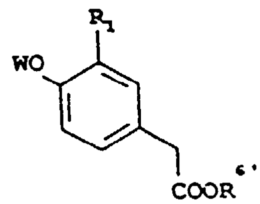

(IIb)

dans laquelle

$R^1$ et $R^{6'}$ ont la signification indiquée ci-dessus,

après élimination du groupe protecteur, d'abord avec des 2-halogénométhylquinoléines répondant à la formule générale (III) dans des solvants inertes par éthérification pour obtenir les composés répondant à la formule générale (IVb)

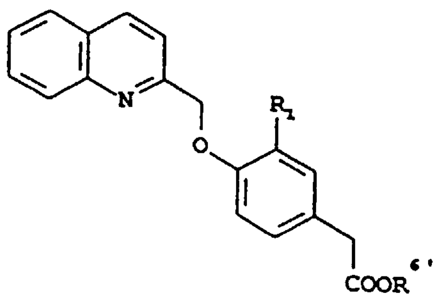

(IVb)

dans laquelle

$R^1$ et $R^{6'}$ ont la signification indiquée ci-dessus,

et ensuite on soumet ces derniers à une alkylation avec des composés répondant à la formule générale (V)

$$R^2\text{-}Z \qquad (V)$$

dans laquelle

$R^2$ a la signification indiquée ci-dessus

et

Z représente un atome de chlore, un atome de brome ou un atome d'iode,

dans des solvants inertes, et dans le cas des esters ($R^6 \neq H$), on soumet les esters à une saponification, ou bien

[B] dans le cas où $R^3$ représente le groupe $-NR^7-SO_2R^8$, on soumet à une amidation des composés répondant à la formule générale (IVc)

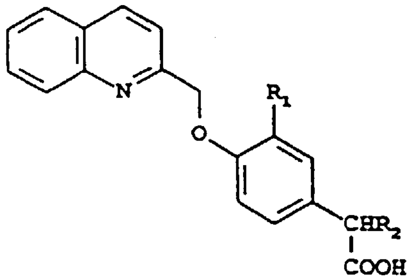

(IVc)

dans laquelle

$R^1$ et $R^2$ ont la signification indiquée ci-dessus,

dans des solvants inertes, éventuellement en présence d'une base, avec des sulfonamides répondant à la formule générale (VI)

$$HNR^7\text{-}SO_2R^8 \quad (VI)$$

dans laquelle

$R^7$ et $R^8$ ont la signification indiquée ci-dessus, et

[C] dans le cas des énantiomères, on sépare les acides correspondants (IVc) en leurs formes énantiomères pures et on soumet celles-ci à une mise en réaction ultérieure conformément aux procédés indiqués ci-dessus.